# EUROPEAN PATENT SPECIFICATION

(11) **EP 3 658 569 B1**
(45) Date of publication and mention of the grant of the patent: **31.01.2024**
(21) Application number: 18760074.7
(22) Date of filing: 26.07.2018
(51) Int. Cl.: C07K 5/103

(54) **PROCESS AND INTERMEDIATES FOR SYNTHESIS OF PEPTIDE COMPOUNDS**
VERFAHREN UND ZWISCHENPRODUKTE ZUR SYNTHESE VON PEPTIDVERBINDUNGEN
PROCÉDÉ ET INTERMÉDIAIRES POUR LA SYNTHÈSE DE COMPOSÉS PEPTIDIQUES

(30) Priority: 28.07.2017 US 201762538117 P
(43) Date of publication of application: 03.06.2020
(73) Proprietor: Naurex Inc., North Chicago, IL 60064 (US)
(72) Inventor: RACHA, Saibaba, Edison New Jersey 08820 (US)
(74) Representative: Hoffmann Eitle
(86) International application number: PCT/US2018/043931
(87) International publication number: WO 2019/023479

(56) References cited:
- WO-A1-2016/014982
- WO-A1-2017/136348
- WO-A2-2013/063120

## Description

### BACKGROUND

An N-methyl-D-aspartate (NMDA) receptor is a postsynaptic, ionotropic receptor that is responsive to, *inter alia*, the excitatory amino acids glutamate and glycine and the synthetic compound NMDA. The NMDA receptor (NMDAR) appears to control the flow of both divalent and monovalent ions into the postsynaptic neural cell through a receptor associated channel and has drawn particular interest since it appears to be involved in a broad spectrum of CNS disorders. The NMDAR has been implicated, for example, in neurodegenerative disorders including stroke-related brain cell death, convulsive disorders, and learning and memory. NMDAR also plays a central role in modulating normal synaptic transmission, synaptic plasticity, and excitotoxicity in the central nervous system. The NMDAR is further involved in Long-Term Potentiation (LTP), which is the persistent strengthening of neuronal connections that underlie learning and memory. The NMDAR has been associated with other disorders ranging from hypoglycemia and cardiac arrest to epilepsy. In addition, there are preliminary reports indicating involvement of NMDA receptors in the chronic neurodegeneration of Huntington's, Parkinson's, and Alzheimer's diseases. Activation of the NMDA receptor has been shown to be responsible for post-stroke convulsions, and, in certain models of epilepsy, activation of the NMDA receptor has been shown to be necessary for the generation of seizures. In addition, certain properties of NMDA receptors suggest that they may be involved in the information-processing in the brain that underlies consciousness itself. Further, NMDA receptors have also been implicated in certain types of spatial learning.

In view of the association of NMDAR with various disorders and diseases, NMDA-modulating small molecule agonist and antagonist compounds have been developed for therapeutic use. NMDA receptor compounds may exert dual (agonist/antagonist) effect on the NMDA receptor through the allosteric sites. These compounds are typically termed "partial agonists". In the presence of the principal site ligand, a partial agonist will displace some of the ligand and thus decrease Ca⁺⁺ flow through the receptor. In the absence of the principal site ligand or in the presence of a lowered level of the principal site ligand, the partial agonist acts to increase Ca⁺⁺ flow through the receptor channel.

Recently, an improved partial agonist of NMDAR with the following structure has been reported (rapastinel):

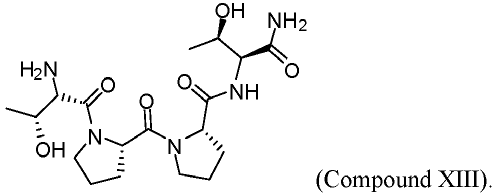

PCT/US2017/015851 (WO 2017/136348 A1) describes a process for synthesis of peptide compounds, including rapastinel. However, a need exists for improved rapastinel (GLYX-13) synthetic methods that, for example, minimize the use of costly and/or toxic reagents, eliminate cumbersome purification steps, are more efficient, result in higher purity rapastinel, and can be utilized in large-scale industrial production of rapastinel.

WO 2016/014982 A1 and WO 2013/063120 A2 disclose NMDAR modulators and methods of making and using the modulators.

### SUMMARY

Disclosed is a new process for preparing dipyrrolidine peptide compounds such as, for example, rapastinel (GLYX-13). Advantageously, the process may be industrially scalable, produce higher yields and improved purity compared to those in the art, and be cost-effective and use less toxic reagents and/or solvents. Further, the process may be used to prepare peptide compounds having improved purity.

In one aspect, a process for synthesizing a dipyrrolidine peptide compound or a pharmaceutically acceptable salt, stereoisomer, metabolite, or hydrate thereof is provided. The process comprises the steps:
a) contacting a compound of Formula III, or a salt thereof: 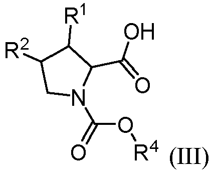 with an activating reagent and a compound of Formula II, or a salt thereof: 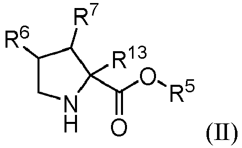 to produce a compound of Formula IV, or a salt thereof: 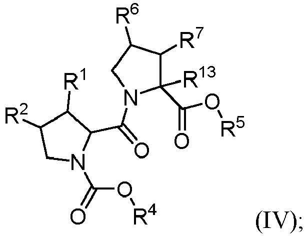
b) contacting the compound of Formula IV, or a salt thereof, with a reagent capable of effecting hydrolysis to produce a compound of Formula V, or a salt thereof: 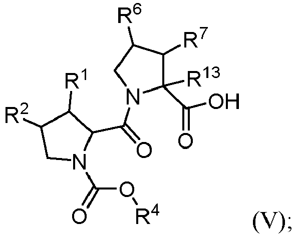
c) contacting the compound of Formula V, or a salt thereof, with an activating reagent and a compound of Formula VIII: 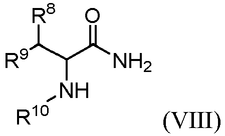 to produce a compound of Formula IX, or a salt thereof: 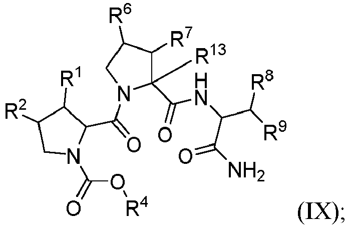
d) contacting the compound of Formula IX, or a salt thereof, with a carbamate-cleaving reagent to produce a compound of Formula XI, or a salt thereof: 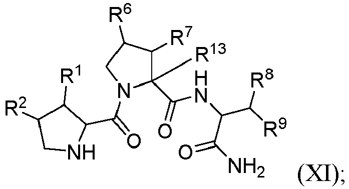
e) contacting a compound of Formula X, or a salt thereof: 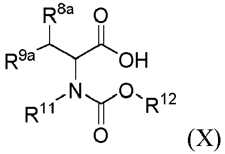 with an activating reagent and the compound of Formula XI, or a salt thereof, to produce a compound of Formula XIIa, or a salt thereof: 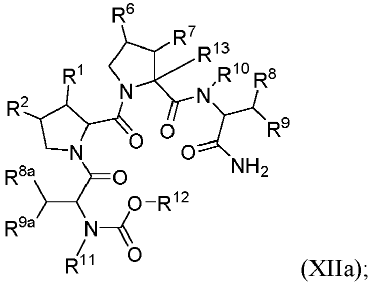
f) contacting the compound of Formula XIIa, or a salt thereof, with a silyl ether cleaving reagent to produce a compound of Formula XIII, or a salt thereof: 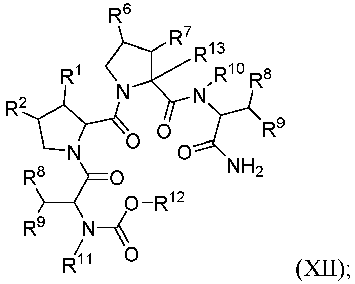 and
g) contacting the compound of Formula XII, or a salt thereof, with a carbamate-cleaving reagent to produce a compound of Formula XIII, or a salt thereof: 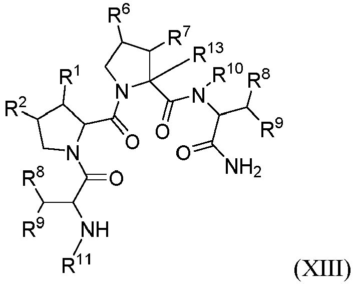 wherein the process further comprises producing the compound of Formula X, or a salt thereof, by
x) contacting a compound of Formula VI, or a salt thereof: 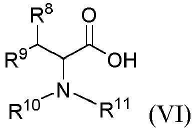 with an activated carbonyl compound to produce a compound of Formula Xa, or a salt thereof: 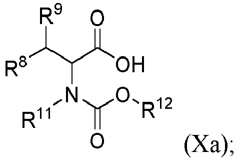 and
y) contacting the compound of Formula Xa, or a salt thereof, with a silyl halide to produce a compound of Formula X, or a salt thereof;
   wherein:
   R¹ and R² are independently selected from hydrogen, halogen, hydroxyl, substituted or unsubstituted C₁₋₆ alkyl, substituted or unsubstituted C₁₋₆ alkoxy, and substituted or unsubstituted aryl, or R¹ and R², together with the atoms to which they are attached, form a substituted or unsubstituted 4-6 membered heterocyclic or cycloalkyl ring;
   R⁴, R⁵, and R¹² are independently -C₁₋₆ alkylene-phenyl, wherein C₁₋₆ alkylene is optionally substituted by one or more substituents each independently selected from R^{f};
   R⁶ and R⁷ are independently selected from hydrogen, halogen, hydroxyl, substituted or unsubstituted C₁₋₆ alkyl, substituted or unsubstituted C₁₋₆ alkoxy, and substituted or unsubstituted aryl, or R⁶ and R⁷, together with the atoms to which they are attached, form a substituted or unsubstituted 4-6 membered heterocyclic or cycloalkyl ring;
   R⁸ and R⁹ are independently selected from hydrogen, halogen, C₁₋₆ alkyl, C₂₋₆ alkenyl, C₂₋₆ alkynyl, C₃₋₆ alkynyl, phenyl, naphthyl, heteroaryl, heterocyclyl, C₃₋₆ cycloalkyl-C₁₋₆ alkyl-, phenyl-C₁₋₆ alkylene-, naphthyl-C₁₋₆ alkylene-, heteroaryl-C₁₋₆ alkylene-, heterocyclyl-C₁₋₆ alkylene-, -OR^{x}, -NO₂, -N₃, -CN, -SCN, -SR^{x}, -C(O)R^{x}, -CO₂(R^{x}), -C(O)N(R^{x})₂, -C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, -NR^{x}C(O)N(R^{x})₂, -NR^{x}C(O)OR^{x}, -NR^{x}C(NR^{x})N(R^{x})₂, and -C(R^{x})₃; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH-moiety, that -NH- moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl and C₁₋₆ alkylene are each independently optionally substituted by one or more substituents each independently selected from R^{f}; and wherein C₃₋₆ cycloalkyl is independently optionally substituted by one or more substituents each independently selected from R^{g};
   R^{8a} and R^{9a} are independently selected from hydrogen, halogen, C₁₋₆ alkyl, C₂₋₆ alkenyl, C₂₋₆ alkynyl, C₃₋₆ cycloalkyl, phenyl, naphthyl, heteroaryl, heterocyclyl, C₃₋₆ cycloalkyl-C₁₋₆ alkyl-, phenyl-C₁₋₆ alkylene-, naphthyl-C₁₋₆ alkylene-, heteroaryl-C₁₋₆ alkylene-, heterocyclyl-C₁₋₆ alkylene-, -OR^{x}, -OR^{y}, -NO₂, -N₃, -CN, -SCN, -SR^{x}, -C(O)R^{x}, -CO₂(R^{x}), -C(O)N(R^{x})₂, - C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, - NR^{x}C(O)N(R^{x})₂, -NR^{x}C(O)OR^{x}, -NR^{x}C(NR^{x})N(R^{x})₂, and -C(R^{x})₃; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH- moiety, that -NH- moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl and C₁₋₆ alkylene are each independently optionally substituted by one or more substituents each independently selected from R^{f}; wherein C₃₋₆ cycloalkyl is independently optionally substituted by one or more substituents each independently selected from R^{g}; and wherein one of R^{8a} or R^{9a} is -OR^{y}.
   R¹⁰ and R¹¹ are independently selected from hydrogen, C₁₋₆ alkyl, -C(O)-C₁₋₆ alkylene, - C(O)-O-C₁₋₆ alkylene, and -C(O)-phenyl, wherein C₁₋₆ alkyl, C₁₋₆ alkylene, and phenyl are optionally independently substituted by one or more substituents selected from R^{a};
   R¹³ is selected from hydrogen and benzyl;
   R^{b} is selected, independently for each occurrence, from halogen, hydroxyl, -NO₂, -N₃, - CN, -SCN, C₁₋₆ alkyl, C₂₋₆ alkenyl, C₂₋₆ alkynyl, C₃₋₆ cycloalkyl, C₁₋₆ alkoxy, C₃₋₆ alkenyloxy, C₃₋₆ alkynyloxy, C₃₋₆ cycloalkoxy, C₁₋₆ alkyl-S(O)_{w}-, where w is 0, 1, or 2, C₁₋₆ alkyl-C₃₋₆ cycloalkyl-, C₃₋₆ cycloalkyl-C₁₋₆ alkyl-, C₁₋₆ alkoxycarbonyl-N(R^{a})-, C₁₋₆ alkyl-N(R^{a})-, C₁₋₆ alkyl-N(R^{a})carbonyl-, R^{a}R^{a'}N-, R^{a}R^{a}'N-carbonyl-, RaR^{a'}N-carbonyl-N(R^{a})-, R^{a}R^{a'}N-SO₂-, and C₁₋₆ alkyl-carbonyl-N(R^{a})-;
   R^{a} and R^{a'} are selected, independently for each occurrence, from hydrogen and C₁₋₆ alkyl, or R^{a} and R^{a'}, when taken together with the nitrogen to which they are attached, form a 4-6 membered heterocyclic ring, wherein C₁₋₆ alkyl is optionally substituted by one or more substituents each independently selected from halogen, oxo, and hydroxyl, and wherein the heterocyclic ring is optionally substituted by one or more substituents each independently selected from halogen, alkyl, oxo, and hydroxyl;
   R^{c} is selected, independently for each occurrence, from halogen, hydroxyl, -NO₂, -N₃, - CN, -SCN, oxo, C₁₋₆ alkyl, C₂₋₆ alkenyl, C₂₋₆ alkenyl, C₃₋₆ cycloalkyl, C₁₋₆ alkoxy, C₃₋₆ alkenyloxy, C₃₋₆ alkynyloxy, C₃₋₆ cycloalkoxy, C₁₋₆ alkyl-S(O)_{w}-, where w is 0, 1, or 2, C₁₋₆ alkyl-C₃₋₆ cycloalkyl-, C₃₋₆ cycloalkyl-C₁₋₆ alkyl-, C₁₋₆ alkoxycarbonyl-N(R^{a})-, C₁₋₆ alkyl-N(R^{a})-, C₁₋₆ alkyl-N(R^{a})carbonyl-, R^{a}R^{a'}N-, R^{a}R^{a'}N-carbonyl-, R^{a}R^{a'}N-carbonyl-N(R^{a})-, R^{a}R^{a'}N-SO₂-, and C₁₋₆ alkyl-carbonyl-N(R^{a})-;
   R^{d} is selected, independently for each occurrence, from C₁₋₆ alkyl, C₁₋₆ alkylcarbonyl, and C₁₋₆ alkylsulfonyl, wherein C₁₋₆ alkyl is optionally substituted by one or more substituents each independently selected from halogen, hydroxyl, and R^{a}R^{a'}N-;
   R^{e} is selected, independently for each occurrence, from halogen, hydroxyl, -NO₂, -N₃, - CN, -SCN, C₁₋₄ alkoxy, C₁₋₄ alkoxycarbonyl, R^{a}R^{a'}N-, R^{a}R^{a}'N-carbonyl, R^{a}R^{a'}N-SO₂-, and C₁₋₄ alkylS(O)_{w}-, where w is 0, 1, or 2;
   R^{f} is selected, independently for each occurrence, from halogen, hydroxyl, -NO₂, -N₃, - CN, -SCN, C₁₋₄ alkoxy, C₁₋₄ alkoxycarbonyl, R^{a}R^{a'}N-, R^{a}R^{a'}N-carbonyl, R^{a}R^{a'}N-SO₂-, and C₁₋₄ alkylS(O)_{w}-, where w is 0, 1, or 2;
   R^{g} is selected, independently for each occurrence, from halogen, hydroxyl, -NO₂, -N₃, - CN, -SCN, C₁₋₆ alkyl, C₁₋₄ alkoxy, C₁₋₄ alkoxycarbonyl, R^{a}R^{a'} N-, R^{a}R^{a'}N-carbonyl, R^{a}R^{a'}N-SO₂-, and C₁₋₄ alkylS(O)_{w}-, where w is 0, 1, or 2;
   R^{x} is selected, independently, from hydrogen, halogen, C₁₋₆ alkyl, C₂₋₆ alkenyl, C₂₋₆ alkynyl, C₃₋₆ cycloalkyl, phenyl, naphthyl, heteroaryl, heterocyclyl, C₃₋₆ cycloalkyl-C₁₋₆ alkyl-, phenyl-C₁₋₆ alkyl-, naphthyl-C₁₋₆ alkyl-, heteroaryl-C₁₋₆ alkyl-, and heterocyclyl-C₁₋₆ alkyl-; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH- moiety, that -NH-moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl is optionally substituted by one or more substituents each independently selected from R^{f}; and wherein C₃₋₆ cycloalkyl is independently optionally substituted by one or more substituents each independently selected from R^{g}; and
   R^{y} is selected, independently, from alkylsilyl, arylsilyl and heteroarylsilyl, wherein the heteroaryl of heteroarylsilyl is a 5-6 membered ring having one, two, or three heteroatoms each independently selected from N, O, and S and optionally substituted with one or more substituents each independently selected from R^{b}; and wherein the alkyl or aryl of the alkylsilyl or arylsilyl, respectively, is optionally substituted by one or more substituents each independently selected from R^{f}.

In some embodiments, step (a) is carried out at a temperature between about -10 °C and about 10 °C. In some embodiments, step (b) is carried out at a temperature between about 15 °C and about 30 °C. In some embodiments, step (c) is carried out at a temperature between about 0 °C and about 30 °C. In some embodiments, step (d) is carried out at a temperature between about 15 °C and about 30 °C. In some embodiments, step (e) is carried out at a temperature between about -10 °C and about 30 °C. In some embodiments, step (f) is carried out at a temperature between about 15 °C and about 30 °C.

In certain embodiments, the compound of Formula VIII is produced by the steps:
h) contacting a compound represented by Formula VI: 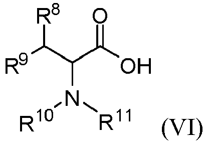 with an activating reagent to form a compound represented by Formula VII: 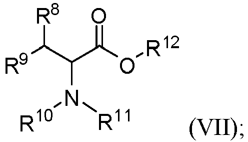 and
i) contacting the compound of Formula VII with an amine to produce the compound of Formula VIII. In some instances, the amine is NH₃. In some embodiments, step (h) is carried out at a temperature between about -10 °C and about 100 °C. In some embodiments, step (i) is carried out at a temperature between about 15 °C and about 30 °C.

In some cases, the compound of Formula II is produced by contacting a compound of Formula I: 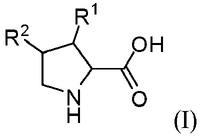 with an activating reagent and an alcohol. In some embodiments, producing the compound of Formula II is carried out at a temperature of between about 0 °C to about 100 °C. In other embodiments, producing the compound of Formula II is carried out at a temperature of between about 0 °C to about 5 °C.

In some embodiments, the compound of Formula III is produced by contacting the compound of Formula II with an activated carbonyl reagent and a base. In some embodiments, the process further comprises contacting the compound of Formula VI with a base. In some instances, the base is NaHCO₃.

In some embodiments, the activating reagent comprises SOCl₂. In some instances, the alcohol is MeOH. In some embodiments, the activated carbonyl reagent is Cbz-Cl. In some cases, the base is a hydroxide salt. In some embodiments, the reagent capable of effecting hydrolysis comprises LiOH. For example, the reagent capable of effecting hydrolysis of the compound of Formula IV comprises LiOH. In some cases, the activating reagent comprises 1-ethyl-3-(3-dimethyllaminopropyl)carbodiimide. In some embodiments, the carbamate-cleaving reagent comprises palladium on carbon.

In some embodiments, the compound of Formula III is produced by contacting the compound of Formula I with an activating reagent and an alcohol to produce a reaction mixture comprising a compound of Formula II, and the reaction mixture is contacted with an activated carbonyl reagent and a base to produce the compound of Formula III.

### BRIEF DESCRIPTION OF DRAWINGS

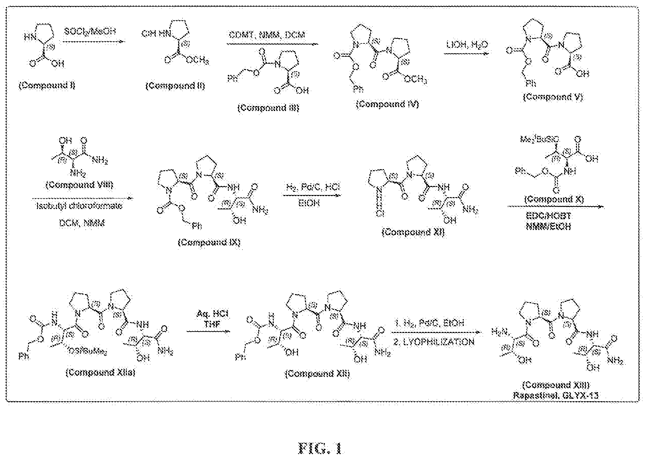

FIG. 1 is a schematic of a synthesis process for rapastinel according to an embodiment of this invention.

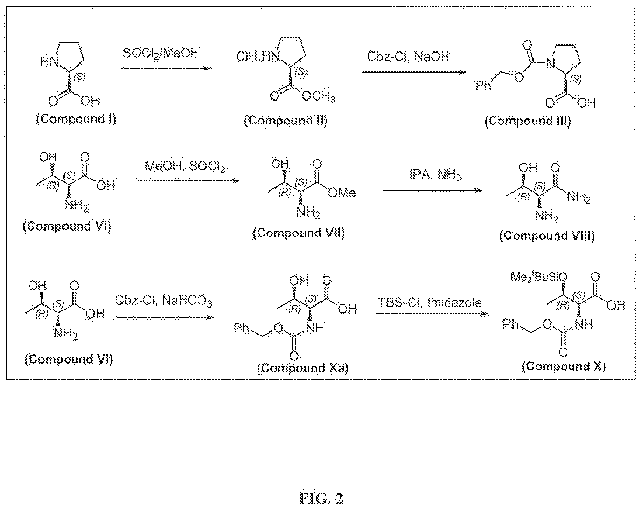

FIG. 2 is a schematic of synthesis processes for Compounds III, VIII and X according to another embodiment of this invention.

### DETAILED DESCRIPTION

Described herein is a new process for preparing dipyrrolidine peptide compounds. As a non-limiting example, the process may be used to prepare rapastinel or analogs or intermediates thereof. Advantageously, the process described herein may be used to prepare dipyrrolidine peptide compounds with higher purity and/or at less cost than known processes. Additionally, less toxic reagents and/or minimalist downstream processes may be used in contrast to known processes. Further, process may be scaled to produce industrial quantities of dipyrrolidine peptide compounds, e.g., greater than 1 kg of compound.

In some embodiments, the steps of the process may be carried out without using N-hydroxybenzotriazole (HOBT) and/or dichloromethane. This aspect may be advantageous since both HOBT and dichloromethane are costly raw materials, which increases the final process costs. Further, rapastinel is soluble in HOBT and the separation of this reaction mixture can be difficult. Consequently, the final purity of rapastinel may be compromised. Additionally, HOBT and dichloromethane are known to be toxic compounds, so their use introduces or increases the toxicity levels of the process. Of course, increased toxicity can result in increased process costs, for example, due to increased costs of handling toxic materials, increased waste disposal costs, and more expensive purification steps.

It will be appreciated by those of ordinary skill in the art that each of the embodiments contemplated herein may be utilized individually or combined in one or more manners different that the ones disclosed herein to produce an improved process for the production of dipyrrolidine peptide compounds. One skilled in the art will be able to select a suitable temperature and other such parameters in view of the reaction conditions being used in different embodiments.

### Processes

The invention provides a process for preparing a compound of Formula XIII or a pharmaceutically acceptable salt, stereoisomer, metabolite, or hydrate thereof, as defined above.

In one embodiment, at least one of R^{8a} and R^{9a} is selected from -OR^{y}.

In some embodiments, R¹ and R² may be hydrogen. In certain embodiments, R⁶ and R⁷ may be hydrogen. In some instances, R¹⁰ and/or R¹¹ may be hydrogen.

In some embodiments, one of R⁸ or R⁹ may be hydrogen.

In some embodiments, R¹³ is hydrogen.

In certain embodiments, the compound of Formula IV may be

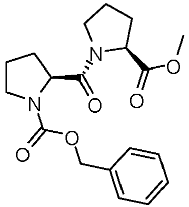

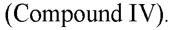

The compound of Formula V may be, for example,

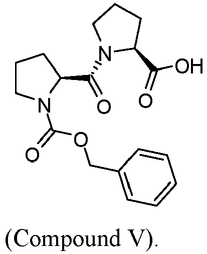

One non-limiting example of a compound of Formula VIII is 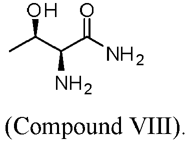 A compound of Formula IX may be exemplified by 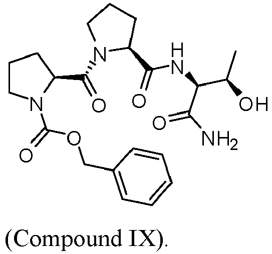 In some embodiments, a compound of Formula X may be 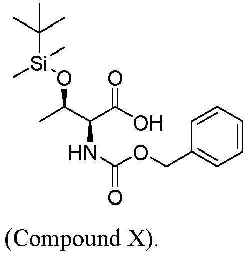 In some cases, a compound of Formula XI may be 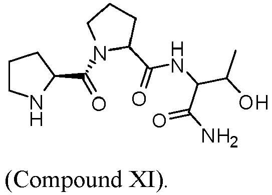 One non-limiting example of a compound of Formula XIIa is

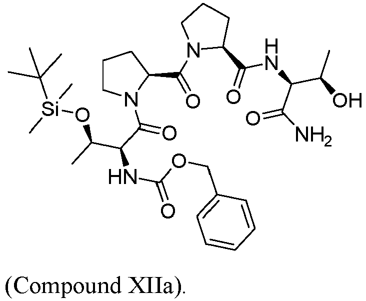

In certain embodiments, a base may be included in the reaction between the compound of the Formula VI and the activated carbonyl compound.

An activating agent may be any reagent capable of activating a carboxyl group for nucleophilic substitution. For example, in some embodiments, the activating agent may be used to convert the carboxyl group to an acyl halide, which may then undergo nucleophilic substitution. For instance, the reagent SOCl₂ may be used to convert the carboxyl group to an acyl chloride. In another embodiment, a carbodiimide may be used to activate a carboxyl group. For example, 1-ethyl-3-(3-dimethyllaminopropyl)carbodiimide (i.e., EDC), N,N'-dicyclohexylcarbodiimide (i.e., DCC), or N,N'-diisopropylcarbodiimide (i.e., DIC) may be used. In some embodiments, a carbodiimide-activated carboxyl group may be reacted to form an activated carbonyl group having more stability than a carbodiimide-activated carboxyl group. For example, the carbodiimide-activated carboxyl group may be reacted with N-hydroxysuccinimide or a suitable alternative thereof to form a less labile activated carbonyl group. In some embodiments, a chlorotriazine may be used to activate a carboxyl group. For example, 2-chloro-4,6-dimethoxy-1,3,5-triazine (i.e., CDMT), or 2,4,6-trichloro-1,3,5-triazine (TCT) may be used. In some embodiments, an alkyl chloroformate may be used to activate a carboxyl group. For example, methyl, ethyl or isobutyl chloroformates may be used.

An activated carbonyl compound may be reacted with a nucleophile to form, for example, an ester or amide. For example, in some embodiments, the activated carbonyl compound may be reacted with an alcohol (e.g., methanol, ethanol, or any other suitable alcohol) to form, for example, an ester or carbonate. In other embodiments, the activated carbonyl may be reacted with an amine to form, for example, an amide or carbamate. In one embodiment, the activated carbonyl compound may be a compound capable of forming a hydrogenation-labile carbonate or carbamate, e.g., benzyl chloroformate (i.e., Cbz-Cl).

In certain embodiments, reaction of an activated carbonyl compound with a nucleophile generates acid as a byproduct. For example, reaction of an acyl chloride with an alcohol or amine generates hydrochloric acid. In certain embodiments, it may be desirable to include a suitable acid scavenger in an acylation reaction. For example, a base such as a hydroxide salt (e.g., lithium hydroxide, sodium hydroxide, and the like), a carbonate (e.g., sodium carbonate, calcium carbonate, magnesium carbonate, and the like), or a bicarbonate (e.g., sodium bicarbonate) may be used.

A reagent capable of effecting hydrolysis may be any suitable reagent having this property. For example, the reagent may be a base such as a hydroxide salt (e.g., lithium hydroxide, sodium hydroxide, and the like).

A carbamate-cleaving reagent may be any suitable reagent capable of liberating an amine from a carbamate. The reagent may be chosen, for example, based on the identity of the carbamate. For instance, a base (e.g., a hydroxide salt) may be used to hydrolyze a carbamate. In embodiments where the carbamate comprises an alkyl-aryl ester (e.g., a benzyl ester), the carbamate-cleaving reagent may be a catalytic hydrogenation reagent (e.g., palladium on carbon (Pd/C)).

A silyl ether cleaving reagent may be any suitable reagent capable of liberating an alcohol from a silyl ether. The reagent may be chosen, for example, based on the identity of the silyl ether. For instance, an acid (e.g., HCl) or a fluoride (e.g., tetrabutyl ammonium fluoride) may be used to cleave a silyl ether.

Each of the steps of the processes contemplated herein may be performed at any suitable temperature or gradient of temperatures. For example, a reaction may be carried out at a temperature of between about -20 °C to about 150 °C, in some embodiments about 0 °C to about 100 °C, in some embodiments between 15 °C and about 30 °C, in some embodiments between about -10 °C to about 30 °C, in some embodiments between about -20 °C to about 0 °C, in some embodiments between about 0 °C to about 30 °C, in some embodiments between about 0 °C to about 5 °C, and in some embodiments between about 20 °C to about 30 °C.

In certain embodiments, a lyophilization step may be included in the process. For example, the compound of Formula XIII may be lyophilized. Lyophilizing may be carried out at any suitable temperature or gradient of temperatures. For example, the lyophilization may be carried out at a temperature of between about -50 °C to about 25 °C. In some instances, the temperature may be increased from a first temperature of about -60 °C to about -40 °C to a second temperature of about 15 °C to about 30 °C. The temperature gradient may occur over any suitable period of time. For example, in some embodiments, the period of time may be about 4 to about 200 hours, or may be about 4 to about 48 hours, in some embodiments about 12 to about 36 hours, or in some embodiments about 20 to about 30 hours.

### Definitions

In some embodiments, the compounds, as described herein, may be substituted with any number of substituents or functional moieties. In general, the term "substituted" whether preceded by the term "optionally" or not, and substituents contained in formulas, refer to the replacement of hydrogen radicals in a given structure with the radical of a specified substituent.

In some instances, when more than one position in any given structure may be substituted with more than one substituent selected from a specified group, the substituent may be either the same or different at every position.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. In some embodiments, heteroatoms such as nitrogen may have hydrogen substituents and/or any permissible substituents of organic compounds described herein which satisfy the valencies of the heteroatoms. Non-limiting examples of substituents include acyl; aliphatic; heteroaliphatic; aryl; heteroaryl; arylalkyl; heteroarylalkyl; alkoxy; cycloalkoxy; heterocyclylalkoxy; heterocyclyloxy; heterocyclyloxyalkyl; alkenyloxy; alkynyloxy; aryloxy; heteroalkoxy; heteroaryloxy; alkylthio; arylthio; heteroarylthio; oxo; -F; -Cl; -Br; -I; -OH; -NO₂; -N₃; -CN; -SCN; -SR^{x}; -CF₃; -CH₂CF₃; -CHCl₂; -CH₂OH; -CH₂CH₂OH; -CH₂NH₂; -CH₂SO₂CH₃; -OR^{x}, -C(O)R^{x}; -CO₂(R^{x}); - C(O)N(R^{x})₂; -C(NR^{x})N(R^{x})₂; -OC(O)R^{x}; -OCO₂R^{x}; -OC(O)N(R^{x})₂; -N(R^{x})₂; -SOR^{x}; -S(O)₂R^{x}; - NR^{x}C(O)R^{x}; -NR^{x}C(O)N(R^{x})₂; -NR^{x}C(O)OR^{x}; -NR^{x}C(NR^{x})N(R^{x})₂; and -C(R^{x})₃; wherein each occurrence of R^{x} independently includes, but is not limited to, hydrogen, halogen, acyl, aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted. Furthermore, the compounds described herein are not intended to be limited in any manner by the permissible substituents of organic compounds. In some embodiments, combinations of substituents and variables described herein may be preferably those that result in the formation of stable compounds. The term "stable," as used herein, refers to compounds which possess stability sufficient to allow manufacture and which maintain the integrity of the compound for a sufficient period of time to be detected and preferably for a sufficient period of time to be useful for the purposes detailed herein.

The term "acyl," as used herein, refers to a moiety that includes a carbonyl group. In some embodiments, an acyl group may have a general formula selected from -C(O)R^{x}; -CO₂(R^{x}); -C(O)N(R^{x})₂; -C(NR^{x})N(R^{x})₂; -OC(O)R^{x}; -OCO₂R^{x}; -OC(O)N(R^{x})₂; -NR^{x}C(O)R^{x}; - NR^{x}C(O)N(R^{x})₂; and -NR^{x}C(O)OR^{x}; wherein each occurrence of R^{x} independently includes, but is not limited to, hydrogen, aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted.

The term "aliphatic," as used herein, includes both saturated and unsaturated, straight chain (i.e., unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties.

The term "heteroaliphatic," as used herein, refers to aliphatic moieties that contain one or more oxygen, sulfur, nitrogen, phosphorus, or silicon atoms, e.g., in place of carbon atoms. Heteroaliphatic moieties may be branched, unbranched, cyclic or acyclic and include saturated and unsaturated heterocycles (e.g., morpholino, pyrrolidinyl,), which may be optionally substituted with one or more functional groups or may be unsubstituted.

The terms "aryl" and "heteroaryl," as used herein, refer to mono- or polycyclic unsaturated moieties having preferably 3-14 carbon atoms, each of which may be substituted or unsubstituted. In certain embodiments, "aryl" refers to a mono- or bicyclic carbocyclic ring system having one or two aromatic rings including, but not limited to, phenyl, naphthyl, tetrahydronaphthyl, indanyl, indenyl, and the like. In certain embodiments, "heteroaryl" refers to a mono- or bicyclic heterocyclic ring system having one or two aromatic rings in which one, two, or three ring atoms are heteroatoms independently selected from the group consisting of S, O, and N and the remaining ring atoms are carbon. Non-limiting examples of heteroaryl groups include pyridyl, pyrazinyl, pyrimidinyl, pyrrolyl, pyrazolyl, imidazolyl, thiazolyl, oxazolyl, isooxazolyl, thiadiazolyl,oxadiazolyl, thiophenyl, furanyl, quinolinyl, isoquinolinyl, and the like.

The term "alkenyl" as used herein refers to an unsaturated straight or branched hydrocarbon having at least one carbon-carbon double bond, such as a straight or branched group of 2-12, 2-10, or 2-6 carbon atoms, referred to herein as C₂-C₁₂alkenyl, C₂-C₁₀alkenyl, and C₂₋ C₆alkenyl, respectively. Exemplary alkenyl groups include, but are not limited to, vinyl, allyl, butenyl, pentenyl, hexenyl, butadienyl, pentadienyl, hexadienyl, 2-ethylhexenyl, 2-propyl-2-butenyl, and 4-(2-methyl-3-butene)-pentenyl.

The term "alkenyloxy" used herein refers to a straight or branched alkenyl group attached to an oxygen (alkenyl-O). Exemplary alkenoxy groups include, but are not limited to, groups with an alkenyl group of 3-6 carbon atoms referred to herein as C₃₋₆alkenyloxy. Exemplary "alkenyloxy" groups include, but are not limited to allyloxy and butenyloxy.

The term "alkoxy" as used herein refers to an alkyl group attached to an oxygen (-O-alkyl). Exemplary alkoxy groups include, but are not limited to, groups with an alkyl group of 1-12, 1-8, or 1-6 carbon atoms, referred to herein as C₁-C₁₂alkoxy, C₁-C₈alkoxy, and C₁-C₆alkoxy, respectively. Exemplary alkoxy groups include, but are not limited to methoxy and ethoxy. Similarly, exemplary "alkenoxy" groups include, but are not limited to vinyloxy, allyloxy, and butenoxy.

The term "alkoxycarbonyl" as used herein refers to a straight or branched alkyl group attached to oxygen, attached to a carbonyl group (alkyl-O-C(O)-). Exemplary alkoxycarbonyl groups include, but are not limited to, alkoxycarbonyl groups of 1-6 carbon atoms, referred to herein as C₁₋₆alkoxycarbonyl. Exemplary alkoxycarbonyl groups include, but are not limited to, methoxycarbonyl, ethoxycarbonyl, and t-butoxycarbonyl.

The term "alkynyloxy" used herein refers to a straight or branched alkynyl group attached to an oxygen (alkynyl-O)). Exemplary alkynyloxy groups include, but are not limited to, propynyloxy.

The term "alkyl" as used herein refers to a saturated straight or branched hydrocarbon, for example, such as a straight or branched group of 1-6, 1-4, or 1-3 carbon atom, referred to herein as C₁-C₆alkyl, C₁-C₄alkyl, and C₁-C₃alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, and octyl. For example, alkyl may refer to a C₁₋₆ alkyl, optionally substituted by one, two, or three substituents selected from the group consisting of: halo, nitro, hydroxyl, -NH₂, -NH-alkyl, or alkoxy (e.g. -OCH₃).

The term "alkylcarbonyl" as used herein refers to a straight or branched alkyl group attached to a carbonyl group (alkyl-C(O)-). Exemplary alkylcarbonyl groups include, but are not limited to, alkylcarbonyl groups of 1-6 atoms, referred to herein as C₁-C₆alkylcarbonyl groups. Exemplary alkylcarbonyl groups include, but are not limited to, acetyl, propanoyl, isopropanoyl, and butanoyl.

The term "alkynyl" as used herein refers to an unsaturated straight or branched hydrocarbon having at least one carbon-carbon triple bond, such as a straight or branched group of 2-6, or 3-6 carbon atoms, referred to herein as C₂₋₆alkynyl, and C₃₋₆alkynyl, respectively. Exemplary alkynyl groups include, but are not limited to, ethynyl, propynyl, butynyl, pentynyl, hexynyl, and methylpropynyl.

Alkyl, alkenyl and alkynyl groups can optionally be substituted, if not indicated otherwise, with one or more groups selected from alkoxy, alkyl, cycloalkyl, amino, halogen, and -C(O)alkyl. In certain embodiments, the alkyl, alkenyl, and alkynyl groups are not substituted, i.e., they are unsubstituted.

The term "amide" or "amido" as used herein refers to a radical of the form -R^{a}C(O)N(R^{b})-, -R^{a}C(O)N(R^{b})R^{c}-, or -C(O)NR^{b}R^{c}, wherein R^{a}, R^{b}, and R^{c} are each independently selected from alkoxy, alkyl, alkenyl, alkynyl, amide, amino, aryl, arylalkyl, carbamate, cycloalkyl, ester, ether, formyl, halogen, haloalkyl, heteroaryl, heterocyclyl, hydrogen, hydroxyl, ketone, and nitro. The amide can be attached to another group through the carbon, the nitrogen, R^{b}, R^{c}, or R^{a}. The amide also may be cyclic, for example R^{b} and R^{c}, R^{a} and R^{b}, or R^{a} and R^{c} may be joined to form a 3- to 12-membered ring, such as a 3- to 10-membered ring or a 5- to 6-membered ring. The term "carboxamido" refers to the structure -C(O)NR^{b}R^{c}.

The term "amine" or "amino" as used herein refers to a radical of the form -NR^{d}R^{e}, where R^{d} and R^{e} are independently selected from hydrogen, alkyl, alkenyl, alkynyl, aryl, arylalkyl, cycloalkyl, haloalkyl, heteroaryl, and heterocyclyl. The amino also may be cyclic, for example, R^{d} and R^{e} are joined together with the N to form a 3- to 12-membered ring, e.g., morpholino or piperidinyl. The term amino also includes the corresponding quaternary ammonium salt of any amino group, e.g., -[N(R^{d})(R^{e})(R^{f})]+. Exemplary amino groups include aminoalkyl groups, wherein at least one of R^{d}, R^{e}, or R^{f} is an alkyl group. In certain embodiment, R^{d} and R^{e} are hydrogen or alkyl.

The term "cycloalkoxy" as used herein refers to a cycloalkyl group attached to an oxygen (cycloalkyl-O-).

The term "cycloalkyl" as used herein refers to a monocyclic saturated or partially unsaturated hydrocarbon group of for example 3-6, or 4-6 carbons, referred to herein, e.g., as C₃₋₆cycloalkyl or C₄₋₆cycloalkyl and derived from a cycloalkane. Exemplary cycloalkyl groups include, but are not limited to, cyclohexyl, cyclohexenyl, cyclopentyl, cyclobutyl or, cyclopropyl.

The terms "halo" or "halogen" or "Hal" as used herein refer to F, Cl, Br, or I. The term "haloalkyl" as used herein refers to an alkyl group substituted with one or more halogen atoms.

The terms "heterocyclyl" or "heterocyclic group" are art-recognized and refer to saturated or partially unsaturated 3- to 10-membered ring structures, alternatively 3- to 7-membered rings, whose ring structures include one to four heteroatoms, such as nitrogen, oxygen, and sulfur. Heterocycles may also be mono-, bi-, or other multi-cyclic ring systems. A heterocycle may be fused to one or more aryl, partially unsaturated, or saturated rings. Heterocyclyl groups include, for example, biotinyl, chromenyl, dihydrofuryl, dihydroindolyl, dihydropyranyl, dihydrothienyl, dithiazolyl, homopiperidinyl, imidazolidinyl, isoquinolyl, isothiazolidinyl, isoxazolidinyl, morpholinyl, oxolanyl, oxazolidinyl, phenoxanthenyl, piperazinyl, piperidinyl, pyranyl, pyrazolidinyl, pyrazolinyl, pyridyl, pyrimidinyl, pyrrolidinyl, pyrrolidin-2-onyl, pyrrolinyl, tetrahydrofuryl, tetrahydroisoquinolyl, tetrahydropyranyl, tetrahydroquinolyl, thiazolidinyl, thiolanyl, thiomorpholinyl, thiopyranyl, xanthenyl, lactones, lactams such as azetidinones and pyrrolidinones, sultams, sultones, and the like. The heterocyclic ring may be substituted at one or more positions with substituents such as alkanoyl, alkoxy, alkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl and thiocarbonyl. In certain embodiments, the heterocyclic group is not substituted, i.e., the heterocyclic group is unsubstituted.

The term "heteroaryloxy" refers to a heteroaryl-O- group.

The term "heterocycloalkyl" is art-recognized and refers to a saturated heterocyclyl group as defined above. The term "heterocyclylalkoxy" as used herein refers to a heterocyclyl attached to an alkoxy group. The term "heterocyclyloxyalkyl" refers to a heterocyclyl attached to an oxygen (-O-), which is attached to an alkyl group.

The term "heterocyclylalkoxy" as used herein refers to a heterocyclyl-alkyl-O-group.

The term "heterocyclyloxy" refers to a heterocyclyl-O- group.

The term "heterocyclyloxyalkyl" refers to a heterocyclyl-O-alkyl- group.

The terms "hydroxy" and "hydroxyl" as used herein refers to the radical -OH.

The term "oxo" as used herein refers to the radical =O.

The term "alkylsilyl" refers to one or more alkyl, as defined above, attached to -Si. For example, alkylsilyl may include -SiH₂R, -SiHRR', or -SiRR'R", in which one of R, R' and R" are alkyl groups, which can be the same or different. The groups, -SiH₂CH₃, -SiH(CH₃)₂, -Si(CH₃)₃ and -Si(CH₃)₂C(CH₃)₃, are non-limiting examples of alkylsilyl groups. So long as R is an alkyl group, R' and R" may constitute a non-alkyl group, such as aryl or hereteroaryl. Further, at least one of R, R' and R" may be substituted. For further example, alkylsilyl may refer to a C₁₋₆ alkyl attached to Si, where the C₁₋₆ alkyl is substituted by one, two, or three substituents selected from the group consisting of: halo, nitro, hydroxyl, -NH₂, -NH-alkyl, or alkoxy (e.g. - OCH₃).

The term "arylsilyl" refers to one or more aryl, as defined above, attached to -Si. For example, arylsilyl may include -SiH₂(phenol), -SiH(phenol)₂, or -Si(phenol)₃, where each phenol may be the same or different. Further, arylsilyl may include -Si(aryl)R'R", where, R' and R" may constitute a non-aryl group, such as alkyl or hereteroaryl.

The term "heteroarylsilyl" refers to one or more herteroaryl, as defined above, attached to -Si. For example, heteroarylsilyl may include -SiH₂(heteroaryl), -SiH(heteroaryl)₂, or - Si(heteroaryl)₃, where each heteroaryl may be the same or different. Further, heteroarylsilyl may include -Si(heteroaryl)R'R", where, R' and R" may constitute a non-heteroaryl group, such as alkyl or aryl.

"Pharmaceutically or pharmacologically acceptable" include molecular entities and compositions that do not produce an adverse, allergic or other untoward reaction when administered to an animal, or a human, as appropriate. "For human administration, preparations should meet sterility, pyrogenicity, general safety and purity standards as required by FDA Office of Biologies standards.

As used in the present disclosure, the term "partial NMDA receptor agonist" is defined as a compound that is capable of binding to a glycine binding site of an NMDA receptor; at low concentrations a NMDA receptor agonist acts substantially as agonist and at high concentrations it acts substantially as an antagonist. These concentrations are experimentally determined for each partial agonist.

As used herein "pharmaceutically acceptable carrier" or "excipient" includes any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

For simplicity, chemical moieties that are defined and referred to throughout can be univalent chemical moieties (e.g., alkyl, aryl) or multivalent moieties under the appropriate structural circumstances clear to those skilled in the art. For example, an "alkyl" moiety can be referred to a monovalent radical (e.g. CH₃-CH₂-), or in other instances, a bivalent linking moiety can be "alkyl," in which case those skilled in the art will understand the alkyl to be a divalent radical (e.g., -CH₂--CH₂-), which is equivalent to the term "alkylene." Similarly, in circumstances in which divalent moieties are required and are stated as being "alkoxy", "alkylamino", "aryloxy", "alkylthio", "aryl", "heteroaryl", "heterocyclic", "alkyl" "alkenyl", "alkynyl", "aliphatic", or "cycloalkyl", those skilled in the art will understand that the terms alkoxy", "alkylamino", "aryloxy", "alkylthio", "aryl", "heteroaryl", "heterocyclic", "alkyl", "alkenyl", "alkynyl", "aliphatic", or "cycloalkyl" refer to the corresponding divalent moiety.

The term "compound" as used herein all include pharmaceutically acceptable salts, co-crystals, solvates, hydrates, polymorphs, enantiomers, diastereoisomers, racemates and the like of the compounds.

The term "pharmaceutically acceptable salt(s)" as used herein refers to salts of acidic or basic groups that may be present in compounds used in the present compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including but not limited to malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, *p*-toluenesulfonate and pamoate (i.e., 1,1'-methylene-bis-(2-hydroxy-3-naphthoate)) salts. Compounds included in the present compositions that include an amino moiety may form pharmaceutically acceptable salts with various amino acids, in addition to the acids mentioned above. Compounds included in the present compositions that are acidic in nature are capable of forming base salts with various pharmacologically acceptable cations. Examples of such salts include alkali metal or alkaline earth metal salts and, particularly, calcium, magnesium, sodium, lithium, zinc, potassium, and iron salts. The term "salts" include "pharmaceutically acceptable salts."

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the symbols "R" or "S," depending on the configuration of substituents around the stereogenic carbon atom. The present invention encompasses various stereoisomers of these compounds and mixtures thereof. Stereoisomers include enantiomers and diastereomers. Mixtures of enantiomers or diastereomers may be designated "(±)" in nomenclature, but the skilled artisan will recognize that a structure may denote a chiral center implicitly.

Individual stereoisomers of compounds of the present invention can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns. Stereoisomeric mixtures can also be resolved into their component stereoisomers by well known methods, such as chiral-phase gas chromatography, chiral-phase high performance liquid chromatography, crystallizing the compound as a chiral salt complex, or crystallizing the compound in a chiral solvent. Stereoisomers can also be obtained from stereomerically-pure intermediates, reagents, and catalysts by well known asymmetric synthetic methods.

Geometric isomers can also exist in the compounds of the present invention. The symbol 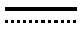 denotes a bond that may be a single, double or triple bond as described herein. The present invention encompasses the various geometric isomers and mixtures thereof resulting from the arrangement of substituents around a carbon-carbon double bond or arrangement of substituents around a carbocyclic ring. Substituents around a carbon-carbon double bond are designated as being in the "*Z*" or "*E*" configuration wherein the terms "*Z*" and "*E*" are used in accordance with IUPAC standards. Unless otherwise specified, structures depicting double bonds encompass both the "*E*" and "*Z*" isomers.

Substituents around a carbon-carbon double bond alternatively can be referred to as "cis" or "trans," where "cis" represents substituents on the same side of the double bond and "trans" represents substituents on opposite sides of the double bond. The arrangement of substituents around a carbocyclic ring are designated as "cis" or "trans." The term "cis" represents substituents on the same side of the plane of the ring and the term "trans" represents substituents on opposite sides of the plane of the ring. Mixtures of compounds wherein the substituents are disposed on both the same and opposite sides of plane of the ring are designated "cis/trans."

The compounds disclosed herein can exist in solvated as well as unsolvated forms with pharmaceutically acceptable solvents such as water, ethanol, and the like, and it is intended that the invention embrace both solvated and unsolvated forms. In one embodiment, the compound is amorphous. In one embodiment, the compound is a polymorph. In another embodiment, the compound is in a crystalline form.

The invention also embraces isotopically labeled compounds of the invention which are identical to those recited herein, except that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

Certain isotopically-labeled disclosed compounds (*e*.*g*., those labeled with ³H and ¹⁴C) are useful in compound and/or substrate tissue distribution assays. Tritiated (*i*.*e*., ³H) and carbon-14 (*i.e.*, ¹⁴C) isotopes are particularly preferred for their ease of preparation and detectability. Further, substitution with heavier isotopes such as deuterium (*i.e.*, ²H) may afford certain therapeutic advantages resulting from greater metabolic stability (*e.g.*, increased *in vivo* half-life or reduced dosage requirements) and hence may be preferred in some circumstances. Isotopically labeled compounds of the invention can generally be prepared by following procedures analogous to those disclosed in the e.g., Examples herein by substituting an isotopically labeled reagent for a non-isotopically labeled reagent.

As used in the present disclosure, "NMDA" is defined as N-methyl-D-aspartate.

### EXAMPLES

The following examples are provided for illustrative purposes only, and are not intended to limit the scope of the disclosure.

### Example 1: Preparation of Methyl L-prolinate hydrochloride (Compound II)

To a solution of L-proline (Compound I, 5 kg, 43.4 moles) in methanol (25 L) was charged thionyl chloride (7.75 kg, 65.1 moles) at 0-5 °C. The reaction mixture was stirred overnight at room temperature and concentrated under reduced pressure below 50 °C. Toluene (5 L) was added and the mixture was distilled and degasified for 2-4 hours under reduced pressure at 50 °C.

¹H-NMR: (500 MHz, DMSO-d6): *δ* 9.09 (s, 1H), 4.35-4.24 (m, 1H), 3.75 (s, 3H), 3.21-3.16 (m, 2H), 2.28-2.21 (m, 2H), 2.01-1.88 (m, 2H).

### Example 2: Preparation of ((Benzyloxy)carbonyl)-L-Proline (Compound III)

A solution of NaOH (6.94 kg, 172.5 moles) in water (35 L) was prepared and added slowly to the reaction mixture obtained from Example 1. The resulting biphasic solution was cooled to 0-5 °C and reacted with a 50% solution of benzyl chloroformate (11.37 L, 47.7 moles) in toluene for 3-4 hours. MTBE (20 L) was added to the reaction mass at 20-30 °C. The reaction mixture was stirred and allowed to settle. After separation of both layers, the aqueous layer was acidified to pH 1-2 and extracted with ethyl acetate. The organic layer was washed with brine solution, dried over sodium sulfate and concentrated under reduced pressure to obtain compound III (10.2 kg, 94% from Compound I).

¹H-NMR (500 MHz, DMSO-*d₆*): *δ* 12.85-12.58 (br s, 1H), 7.38-7.28 (m, 5H), 5.12-4.93 (m, 2H), 4.28 - 4.15 (dd, 1H), 3.45 - 3. 30 (m, 2H), 2.32 - 2.09 (m, 1H), 1.92-1.68 (m, 3H)

### Example 3: Preparation of (S)-Benzyl 2-((S)-2-(methoxycarbonyl) pyrrolidine-1-carbonyl)-pyrrolidine-1-carboxylate (Compound IV)

A solution of Compound I (L-proline, 60 g, 0.52 moles) in methanol (810 mL) was cooled to 0-5 °C and treated with thionyl chloride (100 mL, 1.37 moles). The reaction mixture was stirred for 6-8 hours at 20-35 °C to obtain Compound II. Reaction mixture was distilled to 1-2 volumes under reduced pressure at below 40°C. A series of toluene additions and distillations were performed under reduced pressure at below 50 °C to 1-2 volumes. Dichloromethane (1.0 L) was then added at 25-35 °C.

In another reactor, the Compound III (100 g, 0.4 moles) was dissolved in dichloromethane (500 mL) at below 20 °C and the resulting solution was cooled to 0-5 °C. N-methyl morpholine (NMM, 57 mL, 0.52 moles) was added slowly to it at 0-5 °C. 2-Chloro-4,6-dimethoxy-1,3,5-triazine (CDMT, 74 g, 0.42 moles) was then added slowly to this and the reaction mixture was stirred overnight at room temperature. N-methyl morpholine (NMM, 88 mL, 0.8 moles) was added slowly to the reaction mixture at 0-5 °C. Above prepared L-proline methyl ester hydrochloride (Compound II) solution in DCM was gradually added to the reaction mixture containing the active ester of Compound III at -10 to 5 °C. The temperature of the coupling reaction was increased to room temperature and the mixture was stirred for 3-4 h. The reaction mass was filtered and washed with DCM (200 mL). (Note: By product HDMT was filtered). Combined organic layer was washed with aq. sodium bicarbonate, water and 6N HCl. The organic layer was distilled under reduced pressure at below 45 °C to 1-2 volumes. THF (500 mL) was added and the mixture was distilled under reduced pressure to 1-2 volumes at below 45 °C. A second lot of THF (500 mL) was added and the mixture was distilled under reduced pressure to 1-2 volumes at below 45 °C to obtain Compound IV (8 kg, 85%)

¹H-NMR (400 MHz, DMSO-*d₆*): *δ* 7.39-7.25 (m, 5H), 5.08 - 4.88 (m, 2H), 4.53 - 4.49 (m, 1H), 4.24 (dq, 1H), 3.70 - 3.64 (m, 1H), 3.57 (s, 3H), 3.55 - 3.38 (m, 3H), 2.27-1.66 (m, 8H).

### Example 4: Preparation of (S)-1-((S)-1-(Benzyloxycarbonyl) pyrrolidine-2-carbonyl) pyrrolidine-2-carboxylic acid (Compound V)

To a mixture of THF (500 mL) and water (500 mL) was charged the solution of Compound IV in THF, obtained from Example 3. Lithium hydroxide (20.2 g, 0.48 moles) was added to the reaction mixture and stirred at room temperature overnight. Reaction mixture was washed with MTBE and pH of aqueous layer was adjusted to 1.0-2.0 with concentrated HCl. The resulting slurry was stirred for 1-2 hours at 0-5 °C and filtered. The filter cake was washed with water and MTBE and then dried to obtain Compound V (122 g, 86%).

¹H-NMR (400 MHz, DMSO-*d₆*): *δ* 7.39-7.25 (m, 5H), 5.08 - 4.88 (m, 2H), 4.53 - 4.49 (m, 1H), 4.24 (dq, 1H), 3.70 - 3.64 (m, 1H), 3.57 (s, 3H), 3.55 - 3.38 (m, 3H), 2.27-1.66 (m, 8H).

### Example 5: Preparation of Methyl L-threoninate (Compound VII)

To a cooled solution of Compound VI (L-threonine, 5 kg, 16.7 moles) in methanol (25 L) was added thionyl chloride (7.45 kg, 25 moles) and the reaction mixture was stirred overnight at 20-25 °C. It was then concentrated to a residue and dissolved in methanol (5 L). The methanolic solution was again concentrated to a residue under reduced pressure at below 50 °C and degasified to obtain Compound VII.

### Example 6: Preparation of (2S, 3R)-2-Amino-3-hydroxybutanamide (Compound VIII)

Isopropanol (35 L) was added to the reaction mixture obtained from Example 5. The resulting solution was charged into an autoclave and ammonia gas pressure (4.5-5 Kg) was applied to the reaction mixture at room temperature overnight. It was then filtered, washed with isopropanol (10 L) and filtrate was distilled under reduced pressure to a residue. MTBE (15+5 L) was added slowly and the resulting slurry was stirred and filtered. The filtered cake was dried to obtain Compound VIII (3 kg, 70% from Compound VI).

¹H-NMR: (500 MHz, DMSO-*d₆*): *δ* 7.37 (brs, 1H), 7.02 (brs, 1H), 3.75 (q, 1H), 2.95 (d, 1H), 1.05 (d, 3H).

### Example 7: Preparation of (S)-Benzyl 2-((S)-2-((2S, 3R)-1-amino-3-hydroxy-1-oxobutan-2-ylcarbamoyl) pyrrolidine-1-carbonyl) pyrrolidine-1-carboxylate (Compound IX)

To a cooled solution of Compound V (10 g, 28.87 mmol) in dichloromethane (100 mL) were charged N-methyl morpholine (3.21 g, 31.76 mmol) and isobutylchloroformate (4.33 g, 31.76 mmol). After stirring the mixture for 1-2 h, a second lot of N-methyl morpholine (3.21 g, 31.76 mmol) was added. Compound VIII (4.43 g, 37.52 mmol) was then added and the reaction mixture was left with stirring overnight at room temperature. Brine (30 mL) was charged into the reaction mass, separated the layers and the aqueous layer was extracted with dichloromethane (50 mL). Organic layers were combined and washed with brine solution. The organic layer was concentrated under reduced pressure. The reaction mixture was charged with isopropanol (100 mL) and concentrated to 30-40 mL volume under vacuum. Isopropanol (100 mL) was added and concentrated to 30-40 mL volume under vacuum. The mixture was cooled and the product (Compound IX) was filtered, washed with pre-cooled isopropanol and dried (10.3 g, 78%).

¹H-NMR: (500 MHz, DMSO-*d₆*): *δ* 7.40 - 7. 25 (m, 5H), 7.15 - 7.02 (d, 2H), 5.12 - 4.82 (m, 3H), 4.60 - 4.52 (m, 1H), 4.42 - 4.30 (dd, 1H), 4.08 - 3.96 (m, 2H), 3.70 - 3.33 (m, 4H), 2.30 - 1.60 (m, 8H), 1.05(d, 3H).

### Example 7a: Preparation of (S)-Benzyl 2-((S)-2-((2S, 3R)-1-amino-3-hydroxy-1-oxobutan-2-ylcarbamoyl) pyrrolidine-1-carbonyl) pyrrolidine-1-carboxylate (Compound IX)

To a slurry of Compound VIII.HCl (27.8 g, 0.18 mol) in dichloromethane (900 mL) was added triethylamine (42.5 g, 0.42 mol). The resulting mixture was stirred at room temperature for 1-2 hours. Compound V (50 g, 0.14 mol) was then introduced in one portion. To this, a solution of isobutylchloroformate (20.5 g, 0.15 mol) in dichloromethane (100 mL) was charged at room temperature over 6 hours. The reaction mixture was then left with stirring for 3-4 hours at room temperature. Brine (2 x 250 mL) was charged into the reaction mass, layers were separated and the aqueous layer was extracted with dichloromethane (100 mL). The combined organic layer was concentrated under reduced pressure. The reaction mixture was charged with isopropanol (500 mL) and concentrated to 200-250 mL volume under vacuum. Isopropanol (500 mL) was added and concentrated to 200-250 mL volume under vacuum. The mixture was cooled and the product (Compound IX) was filtered, washed with pre-cooled isopropanol and dried (55.4 g, 86%).

### Example 8: Preparation of ((Benzyloxy)carbonyl)-L-threonine (Compound Xa)

To a mixture of sodium bicarbonate (28.18 kg, 336 moles) and water (50 L) was charged Compound VI (L-threonine, 10 kg, 84 moles). Benzyl chloroformate (31.4 L, 92 moles) was then added at 0-5 °C and the reaction mixture was stirred overnight. After adding MTBE (30 L), the reaction mixture was stirred, layers were separated. The aqueous layer was washed with toluene (20 L) and MTBE (20 L) and then the pH of aqueous layer was adjusted to 1.0-2.0 with concentrated HCl. The reaction mass was stirred for 15 min, then ethyl acetate (30 L) was added. The organic layers were separated and the aqueous layer was extracted with ethyl acetate (20 L). The organic layers were combined and washed with brine solution. Ethyl acetate (100 L) was added to the organic layer. Dicyclohexylamine (30.42 kg, 168 moles) was added and the reaction mixture was stirred at room temperature for 4-5 h. The resulting slurry was cooled, filtered and the filter cake was washed with ethyl acetate (100 L). It was slurried in water (250 L), and the pH was adjusted to 1.0-2.0 with 2N sulphuric acid. The reaction mixture was stirred while ethyl acetate (100 L) was added. The layers were separated and the aqueous layer was extracted with ethyl acetate (100 L). The combined organic layer was dried with sodium sulphate and filtered. The organic layer was concentrated to a residue under vacuum to obtain Compound Xa (9.6 kg, 45%).

¹H-NMR: (500 MHz, DMSO-*d₆*): *δ* 12.75 - 12.55 (brs, 1H), 7.45 - 7.35 (m, 5H), 6.95 (d, 1H), 5.15 (s, 2H), 4.82 - 4.60 (brs, 1H), 4.15 (q, 1H), 3.97 (d, 1H), 1.15 (d, 3H).

### Example 9: Preparation of (2S, 3R)-2-(Benzyloxycarbonylamino)-3-tertiarybutyl dimethyl silyloxy-butanoic acid (Compound X)

A mixture of Compound Xa (25.3 g, 100 mmol), imidazole (14.9 g, 220 moles) and TBDMSCl (16.9 g, 113 mmol) in DMF (21 mL) was heated at 40-50 °C overnight. It was then added slowly with stirring to cold water (375 mL) and the resulting slurry was filtered and washed with cold water (75 ml) and heptane (75 ml). It was then dried under vacuum to obtain Compound X (18 g, 50%).

¹H-NMR: (500 MHz, DMSO-*d₆*): *δ* 12.75 (s, 1H), 7.66 - 7.20 (m, 5H), 6.72 (d, 1H), 5.12 (s, 2H), 4.30 (q, 1H), 4.04 (d, 1H), 1.13 (d, 3H), 0.82 (s, 9H), 0.03 (m, 6H).

### Example 10: Preparation of (S)-N-((2S, 3R)-1-amino-3-hydroxy-1-oxobutan-2-yl)-1-((S)-pyrrolidine-2-carbonyl) pyrrolidine-2-carboxamide (Compound XI)

10% palladium on carbon (w/w, 50% wet; 5.75 g; 0.23 times, w/w) was charged into the pressure reactor at ambient temperature under nitrogen atmosphere. Compound IX (25 g, 56 mmol), ethanol (225 mL) and conc. HCl (5.6 mL, 67.2 mmol) were added to the reactor. Hydrogen pressure was maintained at 45-60 psi at ambient temperature for 5-6 h. Hyflow bed was prepared with ethanol (25 mL). The reaction mass was filtered under nitrogen atmosphere and filter bed was washed with ethanol (25 mL) to obtain Compound XI.

### Example 11: Preparation of Benzyl (2S, 3R)-1-((S)-2-((S)-2-((2S, 3R)-1-amino-3-tertiary butyl dimethyl silyloxy-1-oxobutan-2-ylcarbamoyl) pyrrolidine-1-carbonyl) pyrrolidin-1-yl)-3-hydroxy-1-oxobutan-2-ylcarbamate (Compound Xlla)

An ethanolic solution of Compound XI (56 mmoL), obtained from Example 10, was charged into the reactor under stirring and reaction mixture was cooled to 0-5 °C. EDC.HCl (13.9 g, 72.8 mmol) and HOBT (9.46 g, 61.6 mmol) were charged and the reaction mixture was cooled to -5 to 0 °C. N-methyl morpholine (28.3 g, 280 mmol) was then added dropwise to the above reaction mixture. Compound X (22.64 g, 61.6 mmol) was charged into the reactor under stirring and reaction mixture was left with stirring overnight at room temperature. After completion of the coupling reaction, it was quenched with water (4 volumes) and distilled under vacuum to 3 volumes. Water (7 volumes) and dichloromethane (10 volumes) were charged and both the layers were separated. The organic layer containing coupled product was washed successively with 20% aqueous citric acid solution (10 volumes), water (10 volumes), saturated sodium bicarbonate solution (2 x 10 volumes) and water (10 volumes). It was then concentrated under vacuum at below 50 °C to 3 volumes. From the above reaction, a solution of Compound XIIa in methylene chloride was obtained.

¹H-NMR: (500 MHz, DMSO-*d₆*): *δ* 7.45 - 7.20 (m, 6H), 7.18 - 7. 02 (d, 2H), 5.18 - 4.80 (m, 3H), 4.63 - 4.41 (m, 1H), 4.42 - 4.15 (m, 2H), 4.02 (s, 2H), 3.90 - 3.82 (m, 1H), 3.80 - 3.45 (m, 3H), 2.30 - 1.72 (m, 6H), 1.22 - 1.06 (d, 3H), 1.05 - 0.95 (d, 3H), 1.90 - 1.63 (m, 9H), 0.02 (m, 6H).

### Example 12: Preparation of Benzyl (2S, 3R)-1-((S)-2-((S)-2-((2S, 3R)-1-amino-3-hydroxy-1-oxobutan-2-ylcarbamoyl) pyrrolidine-1-carbonyl) pyrrolidin-1-yl)-3-hydroxy-1-oxobutan-2-ylcarbamate (Compound XIII)

A solution of Compound XIIa (56 mmoL) in methylene chloride, obtained from Example 11, was charged into a reactor. It was diluted with THF (125 mL) and the mixture was distilled under vacuum to 3 volumes (75 ml). A second lot of THF (125 mL) was added and the mixture was distilled under vacuum to 5 volumes (125 mL). It was cooled to 0-5 °C and then water (125 ml) and conc. HCl (9.25 ml) were added. After completion of the reaction (12-16 h), the pH of the mixture was adjusted to 5-6 using aq. sodium bicarbonate solution. Methyl t-butyl ether (250 mL) was charged into the reaction mass. Separated the layers and the aqueous layer was washed with methyl t-butyl ether (250 mL). Brine (50 mL) was charged and the product was extracted with dichloromethane (3 x 75 mL). Organic layers were combined and concentrated to 3 volumes (75 mL) under reduced pressure. The reaction mixture was cooled and charged dropwise to a stirred solution of methyl t-butyl ether (275 mL) to obtain a slurry of the product which was cooled, filtered, and the filter cake was washed with cold methyl t-butyl ether (50 mL) and dried under vacuum to obtain Compound XII (25 g, 85% from Compound XI).

¹H-NMR: (400 MHz, DMSO-*d₆*): *δ* 7.38 - 7. 29 (m, 5H), 7.19 - 7. 17 (d, 1H), 7.08 - 7.04 (d, 1H), 5.03-4.85 (m, 3H), 4.61 - 4.58 (m, 1H), 4.40 - 4.37 (m, 1H), 4.16- 4.12(t, 1H), 4.09-3.99 (m, 2H), 3.84 - 3.58 (m, 4H), 2.32 - 1.72 (m, 8H), 1.11 (d, 3H), 1.00 (d, 3H).

### Example 13: Preparation of Benzyl (S)-N-((2S, 3R)-1-amino-3-hydroxy-1-oxobutan-2-yl)-1-((S)-1-((2R, 3R)-2-amino-3-hydroxybutanoyl) pyrrolidine-2 carbonyl) pyrrolidine-2-carboxamide (rapastinel, Compound XIII)

10% palladium carbon (50% wet; 0.31 kg, 0.11 times, w/w) was charged into the pressure reactor at ambient temperature under nitrogen atmosphere. Compound XII (2.8 kg, 5.11 moles) was dissolved in ethanol (22 L) and added to the reactor. Hydrogen pressure was maintained at 3-4 kg/cm² at ambient temperature over a period of 4-8 hrs. Prepared the hyflow bed (0.28 kg) with ethanol (6 L) and the reaction mass was filtered through hyflow bed under nitrogen atmosphere, and the filtrate was collected into a clean HDPE container. The filter bed was washed with ethanol (15 L) and the filtrate was concentrated under reduced pressure to 2 volumes. The ethanolic solution was charged with dichloromethane (9 L) and stirred till dissolution. MTBE (60 L) was charged to another reactor and stirred. The above EtOH/DCM solution was added to stirred MTBE slowly. During the addition, the product, rapastinel was precipitated. The resulting suspension was filtered, and the filter cake was washed with MTBE (2 x 15 L) and dried under vacuum to obtain rapastinel (2.1 kg, 95%) (Compound XIII).

¹H-NMR: (400 MHz, DMSO-*d₆*): *δ* 7.37 (d, 1H), 7.06 (d, 2H), 4.96 - 4.86 (m, 1H), 4.58 (dd, 1H), 4.45 - 4.34 (m, 1H), 4.07 - 3.99 (m, 2H), 3.70 - 3.65 (m, 2H), 3.61 - 3.54 (m, 2H),3.48 - 3.43 (m, 1H), 3.28 - 3. 22 (m, 1H), 2.32 - 1.68 (m, 8H), 1.07 (d, 3H), 1.00 (d, 3H).

### Example 14: Lyophilization of rapastinel

Rapastinel pure product (3.0 kg, 7.3 moles)) obtained from Stage D was dissolved in water (10 L, 30% w/w)) and stirred for 30 minutes at 20-25 °C. It was then washed with MTBE (2 x 30 L) to remove trace amounts of toluene, ethanol and dichloromethane. The aqueous rapastinel solution is vacuum distilled to remove trace amounts of MTBE. The product rich aqueous solution is polish-filtered. The resulting rapastinel solution is charged into lyophilization trays, freeze-dried until water content is NMT 5.0% w/w, and the final lyophilized rapastinel drug substance (2.8 kg, 95%) is packaged and stored.

## Claims

1. A process for synthesizing a dipyrrolidine peptide compound or a pharmaceutically acceptable salt, stereoisomer, metabolite, or hydrate thereof, comprising the following steps:
a) contacting a compound of Formula III, or a salt thereof: 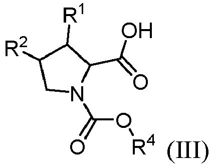 with an activating reagent and a compound of Formula II, or a salt thereof: 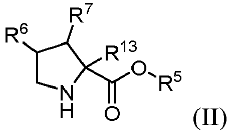 to produce a compound of Formula IV, or a salt thereof: 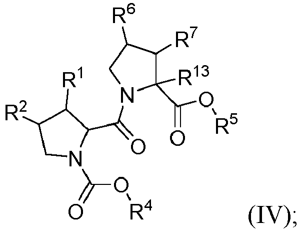
b) contacting the compound of Formula IV, or a salt thereof, with a reagent capable of effecting hydrolysis to produce a compound of Formula V, or a salt thereof: 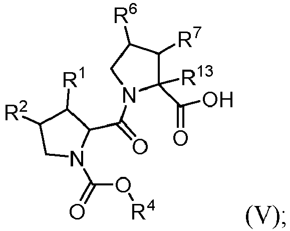
c) contacting the compound of Formula V, or a salt thereof, with an activating reagent and a compound of Formula VIII: 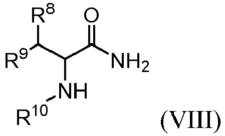 to produce a compound of Formula IX, or a salt thereof: 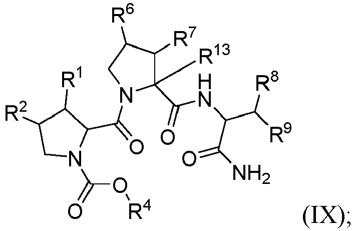
d) contacting the compound of Formula IX, or a salt thereof, with a carbamate-cleaving reagent to produce a compound of Formula XI, or a salt thereof: 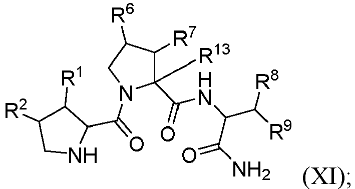
e) contacting a compound of Formula X, or a salt thereof: 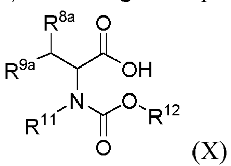 with an activating reagent and the compound of Formula XI, or a salt thereof, to produce a compound of Formula XIIa, or a salt thereof: 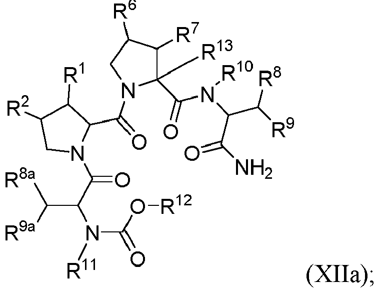
f) contacting the compound of Formula Xlla, or a salt thereof, with a silyl ether cleaving reagent to produce a compound of Formula XII, or a salt thereof: 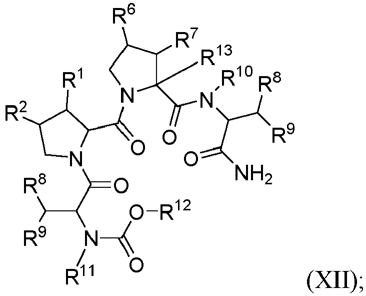 and
g) contacting the compound of Formula XII, or a salt thereof, with a carbamate-cleaving reagent to produce a compound of Formula XIII, or a salt thereof: 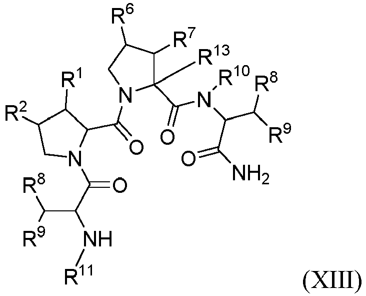 wherein the compound of Formula X, or a salt thereof, is produced by
x) contacting a compound of Formula VI, or a salt thereof: 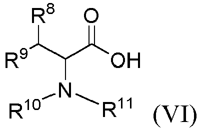 with an activated carbonyl compound to produce a compound of Formula Xa, or a salt thereof: 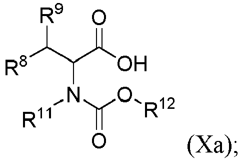 and
y) contacting the compound of Formula Xa, or a salt thereof, with a silyl halide to produce a compound of Formula X, or a salt thereof;
wherein:
R¹ and R² are independently selected from hydrogen, halogen, hydroxyl, substituted or unsubstituted C₁₋₆ alkyl, substituted or unsubstituted C₁₋₆ alkoxy, and substituted or unsubstituted aryl, or R¹ and R², together with the atoms to which they are attached, form a substituted or unsubstituted 4-6 membered heterocyclic or cycloalkyl ring;
R⁴, R⁵, and R¹² are independently -C₁₋₆ alkylene-phenyl, wherein C₁₋₆ alkylene is optionally substituted by one or more substituents each independently selected from R^{f};
R⁶ and R⁷ are independently selected from hydrogen, halogen, hydroxyl, substituted or unsubstituted C₁₋₆ alkyl, substituted or unsubstituted C₁₋₆ alkoxy, and substituted or unsubstituted aryl, or R⁶ and R⁷, together with the atoms to which they are attached, form a substituted or unsubstituted 4-6 membered heterocyclic or cycloalkyl ring;
R⁸ and R⁹ are independently selected from hydrogen, halogen, C₁₋₆ alkyl, C₂₋₆ alkenyl, C₂₋₆ alkynyl, C₃₋₆ alkynyl, phenyl, naphthyl, heteroaryl, heterocyclyl, C₃₋₆ cycloalkyl-C₁₋₆ alkyl-, phenyl-C₁₋₆ alkylene-, naphthyl-C₁₋₆ alkylene-, heteroaryl-C₁₋₆ alkylene-, heterocyclyl-C₁₋₆ alkylene-, -OR^{x}, -NO₂, -N₃, -CN, -SCN, -SR^{x}, -C(O)R^{x}, -CO₂(R^{x}), -C(O)N(R^{x})₂, - C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, -NR^{x}C(O)N(R^{x})₂, -NR^{x}C(O)OR^{x}, -NR^{x}C(NR^{x})N(R^{x})₂, and -C(R^{x})₃; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH- moiety, that -NH- moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl and C₁₋₆ alkylene are each independently optionally substituted by one or more substituents each independently selected from R^{f}; and wherein C₃₋₆ cycloalkyl is independently optionally substituted by one or more substituents each independently selected from R^{g};
R^{8a} and R^{9a} are independently selected from hydrogen, halogen, C₁₋₆ alkyl, C₂₋₆ alkenyl, C₂₋₆ alkynyl, C₃₋₆ cycloalkyl, phenyl, naphthyl, heteroaryl, heterocyclyl, C₃₋₆ cycloalkyl-C₁₋₆ alkyl-, phenyl-C₁₋₆ alkylene-, naphthyl-C₁₋₆ alkylene-, heteroaryl-C₁₋₆ alkylene-, heterocyclyl-C₁₋₆ alkylene-, -OR^{x}, -OR^{y}, -NO₂, -N₃, -CN, -SCN, -SR^{x}, -C(O)R^{x}, -CO₂(R^{x}), -C(O)N(R^{x})₂, - C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, -NR^{x}C(O)N(R^{x})₂, -NR^{x}C(O)OR^{x}, -NR^{x}C(NR^{x})N(R^{x})₂, and -C(R^{x})₃; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH- moiety, that -NH- moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl and C₁₋₆ alkylene are each independently optionally substituted by one or more substituents each independently selected from R^{f}; wherein C₃₋₆ cycloalkyl is independently optionally substituted by one or more substituents each independently selected from R^{g}; and wherein one of R^{8a} or R^{9a} is - OR^{y}.
R¹⁰ and R¹¹ are independently selected from hydrogen, C₁₋₆ alkyl, -C(O)-C₁₋₆ alkylene, -C(O)-O-C₁₋₆ alkylene, and -C(O)-phenyl, wherein C₁₋₆ alkyl, C₁₋₆ alkylene, and phenyl are optionally independently substituted by one or more substituents selected from R^{a};
R¹³ is selected from hydrogen and benzyl;
R^{b} is selected, independently for each occurrence, from halogen, hydroxyl, -NO₂, -N₃, -CN, -SCN, C₁₋₆ alkyl, C₂₋₆ alkenyl, C₂₋₆ alkynyl, C₃₋₆ cycloalkyl, C₁₋₆ alkoxy, C₃₋₆ alkenyloxy, C₃₋₆ alkynyloxy, C₃₋₆ cycloalkoxy, C₁₋₆ alkyl-S(O)_{w}-, where w is 0, 1, or 2, C₁₋₆ alkyl-C₃₋₆ cycloalkyl-, C₃₋₆ cycloalkyl-C₁₋₆ alkyl-, C₁₋₆ alkoxycarbonyl-N(R^{a})-, C₁₋₆ alkyl-N(R^{a})-, C₁₋₆ alkyl-N(R^{a})carbonyl-, R^{a}R^{a'}N-, R^{a}R^{a}'N-carbonyl-, RaR^{a'}N-carbonyl-N(R^{a})-, R^{a}R^{a'}N-SO₂-, and C₁₋₆ alkyl-carbonyl-N(R^{a})-;
R^{a} and R^{a'} are selected, independently for each occurrence, from hydrogen and C₁₋₆ alkyl, or R^{a} and R^{a'}, when taken together with the nitrogen to which they are attached, form a 4-6 membered heterocyclic ring, wherein C₁₋₆ alkyl is optionally substituted by one or more substituents each independently selected from halogen, oxo, and hydroxyl, and wherein the heterocyclic ring is optionally substituted by one or more substituents each independently selected from halogen, alkyl, oxo, and hydroxyl;
R^{c} is selected, independently for each occurrence, from halogen, hydroxyl, -NO₂, -N₃, -CN, -SCN, oxo, C₁₋₆ alkyl, C₂₋₆ alkenyl, C₂₋₆ alkenyl, C₃₋₆ cycloalkyl, C₁₋₆ alkoxy, C₃₋₆ alkenyloxy, C₃₋₆ alkynyloxy, C₃₋₆ cycloalkoxy, C₁₋₆ alkyl-S(O)_{w}-, where w is 0, 1, or 2, C₁₋₆ alkyl-C₃₋₆ cycloalkyl-, C₃₋₆ cycloalkyl-C₁₋₆ alkyl-, C₁₋₆ alkoxycarbonyl-N(R^{a})-, C₁₋₆ alkyl-N(R^{a})-, C₁₋₆ alkyl-N(R^{a})carbonyl-, R^{a}R^{a'}N-, R^{a}R^{a'}N-carbonyl-, RaR^{a'}N-carbonyl-N(R^{a})-, R^{a}R^{a'}N-SO₂-, and C₁₋₆ alkyl-carbonyl-N(R^{a})-;
R^{d} is selected, independently for each occurrence, from C₁₋₆ alkyl, C₁₋₆ alkylcarbonyl, and C₁₋₆ alkylsulfonyl, wherein C₁₋₆ alkyl is optionally substituted by one or more substituents each independently selected from halogen, hydroxyl, and R^{a}R^{a'}N-;
R^{e} is selected, independently for each occurrence, from halogen, hydroxyl, -NO₂, -N₃, -CN, -SCN, C₁₋₄ alkoxy, C₁₋₄ alkoxycarbonyl, R^{a}R^{a'}N-, R^{a}R^{a'}N-carbonyl, R^{a}R^{a'}N-SO₂-, and C₁₋₄ alkylS(O)_{w}-, where w is 0, 1, or 2;
R^{f} is selected, independently for each occurrence, from halogen, hydroxyl, -NO₂, -N₃, -CN, -SCN, C₁₋₄ alkoxy, C₁₋₄ alkoxycarbonyl, R^{a}R^{a'}N-, R^{a}R^{a'}N-carbonyl, R^{a}R^{a'}N-SO₂-, and C₁₋₄ alkylS(O)_{w}-, where w is 0, 1, or 2;
R^{g} is selected, independently for each occurrence, from halogen, hydroxyl, -NO₂, -N₃, -CN, -SCN, C₁₋₆ alkyl, C₁₋₄ alkoxy, C₁₋₄ alkoxycarbonyl, R^{a}R^{a'} N-, R^{a}R^{a'}N-carbonyl, R^{a}R^{a'}N-SO₂-, and C₁₋₄ alkylS(O)_{w}-, where w is 0, 1, or 2;
R^{x} is selected, independently, from hydrogen, halogen, C₁₋₆ alkyl, C₂₋₆ alkenyl, C₂₋₆ alkynyl, C₃₋₆ cycloalkyl, phenyl, naphthyl, heteroaryl, heterocyclyl, C₃₋₆ cycloalkyl-C₁₋₆ alkyl-, phenyl-C₁₋₆ alkyl-, naphthyl-C₁₋₆ alkyl-, heteroaryl-C₁₋₆ alkyl-, and heterocyclyl-C₁₋₆ alkyl-; wherein heteroaryl is a 5-6 membered ring having one, two, or three heteroatoms each independently selected from N, O, and S; wherein heteroaryl is optionally substituted with one or more substituents each independently selected from R^{b}; wherein heterocyclyl is a 4-7 membered ring optionally substituted by one or more substituents each independently selected from R^{c}; wherein when heterocyclyl contains a -NH- moiety, that -NH-moiety is optionally substituted by R^{d}; wherein C₂₋₆ alkenyl and C₂₋₆ alkynyl are each independently optionally substituted by one or more substituents each independently selected from R^{e}; wherein C₁₋₆ alkyl is optionally substituted by one or more substituents each independently selected from R^{f}; and wherein C₃₋₆ cycloalkyl is independently optionally substituted by one or more substituents each independently selected from R^{g}; and
R^{y} is selected, independently, from alkylsilyl, arylsilyl and heteroarylsilyl, wherein the heteroaryl of heteroarylsilyl is a 5-6 membered ring having one, two, or three heteroatoms each independently selected from N, O, and S and optionally substituted with one or more substituents each independently selected from R^{b}; and wherein the alkyl or aryl of the alkylsilyl or arylsilyl, respectively, is optionally substituted by one or more substituents each independently selected from R^{f}.

2. The process of claim 1, wherein the compound of Formula VIII, or a salt thereof, is produced by the following steps:
h) contacting a compound represented by Formula VI, or a salt thereof: 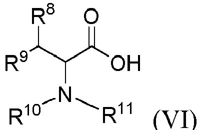 with an activating reagent to form a compound represented by Formula VII, or a salt thereof: 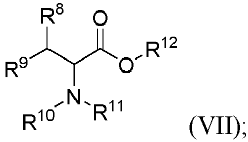 and
i) contacting the compound of Formula VII, or a salt thereof, with an amine to produce the compound of Formula VIII, or a salt thereof.

3. The process of claim 1 or 2, wherein
the compound of Formula II, or a salt thereof, is produced by contacting a compound of Formula I, or a salt thereof: 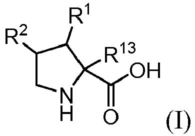 with an activating reagent and an alcohol; and
the compound of Formula III, or a salt thereof, is produced by contacting the compound of Formula II, or a salt thereof, with an activated carbonyl reagent and a base.

4. The process of claim 2 or 3, wherein the activating reagent comprises SOCl₂.

5. The process of claim 3, wherein the alcohol is MeOH.

6. The process of claim 3, wherein the activated carbonyl reagent is Cbz-Cl.

7. The process of claim 3, wherein the base is a hydroxide salt.

8. The process of any preceding claim, wherein the reagent capable of effecting hydrolysis of the compound of Formula IV, or a salt thereof, comprises LiOH.

9. The process of claim 2, wherein the amine is NH₃.

10. The process of any preceding claim, wherein the activating reagent in step (c) comprises 1-ethyl-3-(3-dimethyllaminopropyl)carbodiimide.

11. The process of claim 1, wherein the activated carbonyl compound is Cbz-Cl.

12. The process of claim 1, further comprising contacting the compound of Formula VI, or a salt thereof, with a base.

13. The process of claim 12, wherein the base is NaHCO₃.

14. The process of any preceding claim, wherein the carbamate-cleaving reagent comprises palladium on carbon.

15. The process of any preceding claim wherein the compound of Formula I is 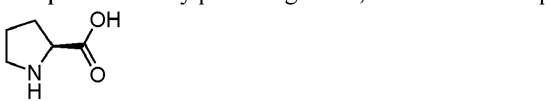 and/or the compound of Formula II is 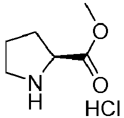 and/or the compound of Formula III is 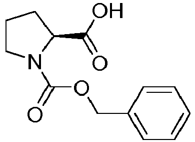 and/or the compound of Formula IV is 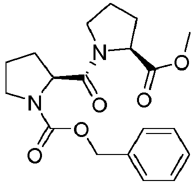 and/or the compound of Formula V is 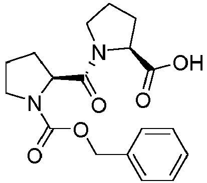 and/or the compound of Formula VI is 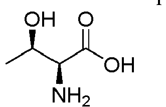 and/or the compound of Formula VII is 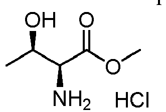 and/or the compound of Formula VIII is 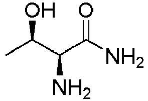 and/or the compound of Formula IX is 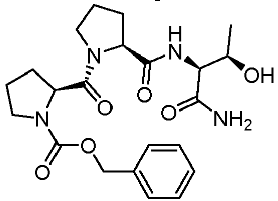 and/or the compound of Formula X is 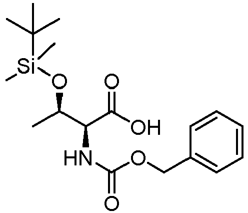 and/or the compound of Formula XI is 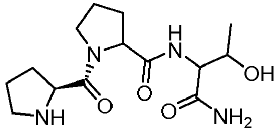 and/or the compound of Formula XIIa 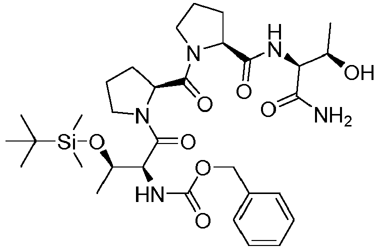 and/or the compound of Formula XIII is

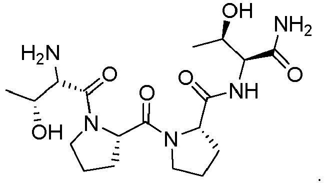

## Patentansprüche

1. Verfahren zur Synthese einer Dipyrrolidin-Peptidverbindung oder eines pharmazeutisch akzeptablen Salzes, Stereoisomers, Metaboliten oder Hydrats davon, umfassend die folgenden Schritte:
a) das Inkontaktbringen einer Verbindung der Formel III oder eines Salzes davon: 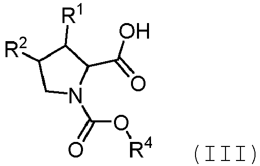 mit einem Aktivierungsreagenz und einer Verbindung der Formel II oder einem Salz davon: 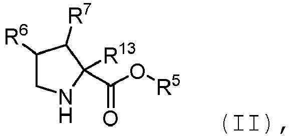 um eine Verbindung der Formel IV oder ein Salz davon herzustellen: 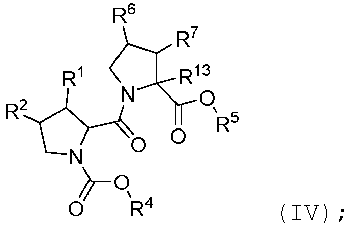
b) das Inkontaktbringen der Verbindung der Formel IV oder eines Salzes davon mit einem Reagenz, das in der Lage ist, eine Hydrolyse zu bewirken, um eine Verbindung der Formel V oder ein Salz davon herzustellen: 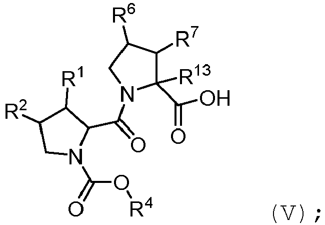
c) das Inkontaktbringen der Verbindung der Formel V oder eines Salzes davon mit einem Aktivierungsreagenz und einer Verbindung der Formel VIII: 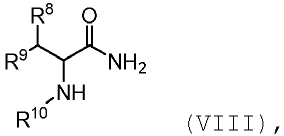 um eine Verbindung der Formel IX oder ein Salz davon herzustellen: 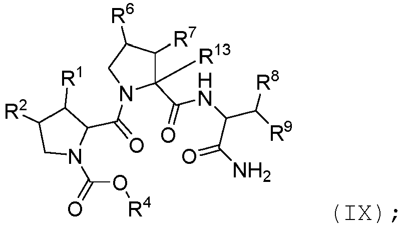
d) das Inkontaktbringen der Verbindung der Formel IX oder eines Salzes davon mit einem Carbamatspaltungsreagenz, um eine Verbindung der Formel XI oder ein Salz davon herzustellen: 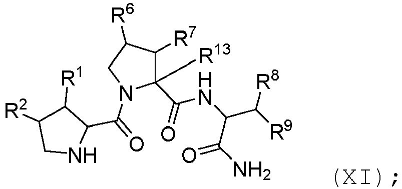
e) das Inkontaktbringen einer Verbindung der Formel X oder eines Salzes davon: 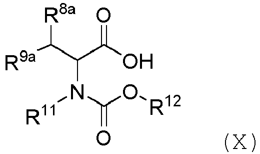 mit einem Aktivierungsreagenz und der Verbindung der Formel XI oder einem Salz davon, um eine Verbindung der Formel XIIa oder ein Salz davon herzustellen: 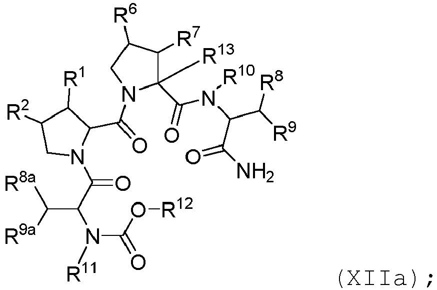
f) das Inkontaktbringen der Verbindung der Formel XIIa oder eines Salzes davon mit einem Silyletherspaltungsreagenz, um eine Verbindung der Formel XII oder ein Salz davon herzustellen: 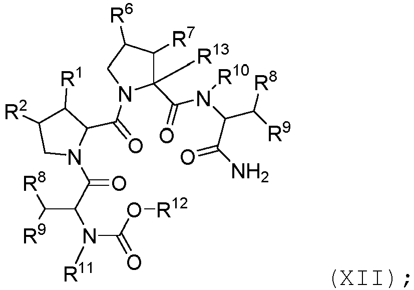 und
g) das Inkontaktbringen der Verbindung der Formel XII oder eines Salzes davon mit einem Carbamatspaltungsreagenz, um eine Verbindung der Formel XIII oder ein Salz davon herzustellen: 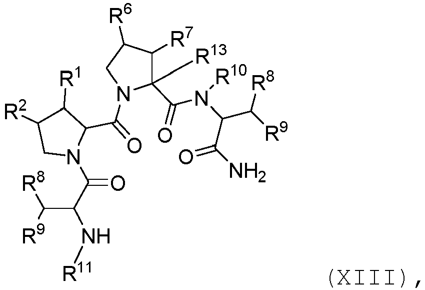 wobei die Verbindung der Formel X oder ein Salz davon hergestellt wird durch
x) das Inkontaktbringen einer Verbindung der Formel VI oder eines Salzes davon: 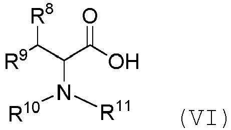 mit einer aktivierten Carbonylverbindung, um eine Verbindung der Formel Xa oder ein Salz davon herzustellen: 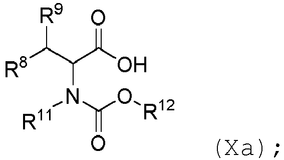 und
y) das Inkontaktbringen der Verbindung der Formel Xa oder eines Salzes davon mit einem Silylhalogenid, um eine Verbindung der Formel X oder ein Salz davon herzustellen;
worin:
R¹ und R² unabhängig voneinander aus Wasserstoff, Halogen, Hydroxyl, substituiertem oder unsubstituiertem C₁₋₆-Alkyl, substituiertem oder unsubstituiertem C₁₋₆-Alkoxy und substituiertem oder unsubstituiertem Aryl ausgewählt sind, oder R¹ und R² zusammen mit den Atomen, an die sie gebunden sind, einen substituierten oder unsubstituierten 4-6-gliedrigen heterocyclischen oder Cycloalkyl-Ring bilden;
R⁴, R⁵ und R¹² unabhängig voneinander -C₁₋₆-Alkylen-Phenyl sind, worin C₁₋₆-Alkylen optional mit einem oder mehreren Substituenten substituiert ist, die jeweils unabhängig voneinander aus R^{f} ausgewählt sind;
R⁶ und R⁷ unabhängig voneinander aus Wasserstoff, Halogen, Hydroxyl, substituiertem oder unsubstituiertem C₁₋₆-Alkyl, substituiertem oder unsubstituiertem C₁₋₆-Alkoxy und substituiertem oder unsubstituiertem Aryl ausgewählt sind, oder R⁶ und R⁷ zusammen mit den Atomen, an die sie gebunden sind, einen substituierten oder unsubstituierten 4-6-gliedrigen heterocyclischen oder Cycloalkyl-Ring bilden;
R⁸ und R⁹ unabhängig voneinander aus Wasserstoff, Halogen, C₁₋₆-Alkyl, C₂₋₆-Alkenyl, C₂₋₆-Alkinyl, C₃₋₆-Alkinyl, Phenyl, Naphthyl, Heteroaryl, Heterocyclyl, C₃₋₆-Cycloalkyl-C₁₋₆-Alkyl-, Phenyl-C₁₋₆-Alkylen-, Naphthyl-C₁₋₆-Alkylen-, Heteroaryl-C₁₋₆-Alkylen-, Heterocyclyl-C₁₋₆-Alkylen-, -OR^{x}, -NO₂, -N₃, -CN, -SCN, -SR^{x}, -C(O)R^{x}, -CO₂(R^{x}), -C(O)N(R^{x})₂, -C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, -NR^{x}C(O)N(R^{x})₂, -NR^{x}C(O)OR^{x}, -NR^{x}C(NR^{x})N(R^{x})₂ und -C(R^{x})₃ ausgewählt sind; wobei Heteroaryl ein 5-6-gliedriger Ring mit einem, zwei oder drei Heteroatomen ist, die jeweils unabhängig voneinander aus N, O und S ausgewählt sind; wobei Heteroaryl optional mit einem oder mehreren Substituenten substituiert ist, die jeweils unabhängig voneinander aus R^{b} ausgewählt sind; wobei Heterocyclyl ein 4-7-gliedriger Ring ist, der optional mit einem oder mehreren Substituenten substituiert ist, die jeweils unabhängig voneinander aus R^{c} ausgewählt sind; wobei, wenn Heterocyclyl eine Gruppe -NH- enthält, diese Gruppe -NH- optional mit R^{d} substituiert ist; wobei C₂₋₆-Alkenyl und C₂₋₆-Alkinyl jeweils unabhängig voneinander optional mit einem oder mehreren Substituenten substituiert sind, die jeweils unabhängig voneinander aus R^{e} ausgewählt sind, wobei C₁₋₆-Alkyl und C₁₋₆-Alkylen jeweils unabhängig voneinander optional mit einem oder mehreren Substituenten substituiert sind, die jeweils unabhängig voneinander aus R^{f} ausgewählt sind; und wobei C₃₋₆-Cycloalkyl unabhängig voneinander optional mit einem oder mehreren Substituenten substituiert ist, die jeweils unabhängig voneinander aus R^{g} ausgewählt sind;
R^{8a} und R^{9a} unabhängig voneinander aus Wasserstoff, Halogen, C₁₋₆-Alkyl, C₂₋₆-Alkenyl, C₂₋₆-Alkinyl, C₃₋₆-Cycloalkyl, Phenyl, Naphthyl, Heteroaryl, Heterocyclyl, C₃₋₆-Cycloalkyl-C₁₋₆-Alkyl-, Phenyl-C₁₋₆-Alkylen-, Naphthyl-C₁₋₆-Alkylen-, Heteroaryl-C₁₋₆-Alkylen-, Heterocyclyl-C₁₋₆-Alkylen-, -OR^{x}, -OR^{y}, -NO₂, -N₃, -CN, -SCN, -SR^{x}, -C(O)R^{x}, -CO₂(R^{x}), -C(O)N(R^{x})₂, -C(NR^{x})N(R^{x})₂, -OC(O)R^{x}, -OCO₂R^{x}, -OC(O)N(R^{x})₂, -N(R^{x})₂, -SOR^{x}, -S(O)₂R^{x}, -NR^{x}C(O)R^{x}, -NR^{x}C(O)N(R^{x})₂, -NR^{x}C(O)OR^{x}, -NR^{x}C(NR^{x})N(R^{x})₂ und -C(R^{x})₃ ausgewählt sind; wobei Heteroaryl ein 5-6-gliedriger Ring mit einem, zwei oder drei Heteroatomen ist, die jeweils unabhängig voneinander aus N, O und S ausgewählt sind; wobei Heteroaryl optional mit einem oder mehreren Substituenten substituiert ist, die jeweils unabhängig voneinander aus R^{b} ausgewählt sind; wobei Heterocyclyl ein 4-7-gliedriger Ring ist, der optional mit einem oder mehreren Substituenten substituiert ist, die jeweils unabhängig voneinander aus R^{c} ausgewählt sind; wobei, wenn Heterocyclyl eine Gruppe -NH- enthält, diese Gruppe -NH- optional mit R^{d} substituiert ist; wobei C₂₋₆-Alkenyl und C₂₋₆-Alkinyl jeweils unabhängig voneinander optional mit einem oder mehreren Substituenten substituiert sind, die jeweils unabhängig voneinander aus R^{e} ausgewählt sind, wobei C₁₋₆-Alkyl und C₁₋₆-Alkylen jeweils unabhängig voneinander optional mit einem oder mehreren Substituenten substituiert sind, die jeweils unabhängig voneinander aus R^{f} ausgewählt sind; und wobei C₃₋₆-Cycloalkyl unabhängig voneinander optional mit einem oder mehreren Substituenten substituiert ist, die jeweils unabhängig voneinander aus R^{g} ausgewählt sind; und wobei eines von R^{8a} oder R^{9a} -ORY ist;
R¹⁰ und R¹¹ unabhängig voneinander aus Wasserstoff, C₁₋₆-Alkyl, -C(O)-C₁₋₆-Alkylen, -C(O)-O-C₁₋₆-Alkylen und -C(O)-Phenyl ausgewählt sind, wobei C₁₋₆-Alkyl, C₁₋₆-Alkylen und Phenyl optional unabhängig voneinander mit einem oder mehreren Substituenten substituiert sind, die aus R^{a} ausgewählt sind;
R¹³ aus Wasserstoff und Benzyl ausgewählt ist;
R^{b} bei jedem Vorkommen unabhängig aus Halogen, Hydroxyl, -NO₂, -N₃, -CN, -SCN, C₁₋₆-Alkyl, C₂₋₆-Alkenyl, C₂₋₆-Alkinyl, C₃₋₆-Cycloalkyl, C₁₋₆-Alkoxy, C₃₋₆-Alkenyloxy, C₃₋₆-Alkinyloxy, C₃₋₆-Cycloalkoxy, C₁₋₆-Alkyl-S(O)_{w}-, worin w 0, 1 oder 2 ist, C₁₋₆-Alkyl-C₃₋₆-Cycloalkyl-, C₃₋₆-Cycloalkyl-C₁₋₆-Alkyl-, C₁₋₆-Alkoxycarbonyl-N(R^{a})-, C₁₋₆-Alkyl-N(R^{a})-, C₁₋₆-Alkyl-N(R^{a})Carbonyl-, R^{a}R^{a'}N-, R^{a}R^{a'}N-Carbonyl-, R^{a}R^{a'}N-Carbonyl-N(R^{a})-, R^{a}R^{a'}N-SO₂- und C₁₋₆-Alkyl-Carbonyl-N(R^{a})- ausgewählt ist;
R^{a} und R^{a'} bei jedem Vorkommen unabhängig voneinander aus Wasserstoff und C₁₋₆-Alkyl ausgewählt sind, oder R^{a} und R^{a'} zusammen mit dem Stickstoff, an den sie gebunden sind, einen 4-6-gliedrigen heterocyclischen Ring bilden, wobei C₁₋₆-Alkyl optional mit einem oder mehreren Substituenten substituiert ist, die jeweils unabhängig voneinander aus Halogen, Oxo und Hydroxyl ausgewählt sind, und wobei der heterocyclische Ring optional mit einem oder mehreren Substituenten substituiert ist, die jeweils unabhängig voneinander aus Halogen, Alkyl, Oxo und Hydroxyl ausgewählt sind;
R^{c} bei jedem Vorkommen unabhängig aus Halogen, Hydroxyl, -NO₂, -N₃, -CN, -SCN, Oxo, C₁₋₆-Alkyl, C₂₋₆-Alkenyl, C₂₋₆-Alkenyl, C₃₋₆-Cycloalkyl, C₁₋₆-Alkoxy, C₃₋₆-Alkenyloxy, C₃₋₆-Alkinyloxy, C₃₋₆-Cycloalkoxy, C₁₋₆-Alkyl-S(O)_{w}-, worin w 0, 1 oder 2 ist, C₁₋₆-Alkyl-C₃₋₆-Cycloalkyl-, C₃₋₆-Cycloalkyl-C₁₋₆-Alkyl-, C₁₋₆-Alkoxycarbonyl-N(R^{a})-, C₁₋₆-Alkyl-N(R^{a})-, C₁₋₆-Alkyl-N(R^{a})Carbonyl-, R^{a}R^{a'}N-, R^{a}R^{a'}N-Carbonyl-, R^{a}R^{a'}N-Carbonyl-N(R^{a})-, R^{a}R^{a'}N-SO₂- und C₁₋₆-Alkyl-Carbonyl-N(R^{a})- ausgewählt ist;
R^{d} bei jedem Vorkommen unabhängig aus C₁₋₆-Alkyl, C₁₋₆-Alkylcarbonyl und C₁₋₆-Alkylsulfonyl ausgewählt ist, wobei C₁₋₆-Alkyl optional mit einem oder mehreren Substituenten substituiert ist, die jeweils unabhängig aus Halogen, Hydroxyl und R^{a}R^{a'}N- ausgewählt sind;
R^{e} bei jedem Vorkommen unabhängig aus Halogen, Hydroxyl, -NO₂, -N₃, -CN, -SCN, C₁₋₄-Alkoxy, C₁₋₄-Alkoxycarbonyl, R^{a}R^{a'}N-, R^{a}R^{a'}N-Carbonyl, R^{a}R^{a'}N-SO₂- und C₁₋₄-AlkylS(O)_{w}-, worin w 0, 1 oder 2 ist, ausgewählt ist;
R^{f} bei jedem Vorkommen unabhängig aus Halogen, Hydroxyl, -NO₂, -N₃, -CN, -SCN, C₁₋₄-Alkoxy, C₁₋₄-Alkoxycarbonyl, R^{a}R^{a'}N-, R^{a}R^{a'}N-Carbonyl, R^{a}R^{a'}N-SO₂- und C₁₋₄-AlkylS(O)_{w}-, worin w 0, 1 oder 2 ist, ausgewählt ist;
R^{g} bei jedem Vorkommen unabhängig aus Halogen, Hydroxyl, -NO₂, -N₃, -CN, -SCN, C₁₋₆-Alkyl, C₁₋₄-Alkoxy, C₁₋₄-Alkoxycarbonyl, R^{a}R^{a'}N-, R^{a}R^{a'}N-Carbonyl, R^{a}R^{a'}N-SO₂- und C₁₋₄-AlkylS(O)_{w}-, worin w 0, 1 oder 2 ist, ausgewählt ist;
R^{x} unabhängig aus Wasserstoff, Halogen, C₁₋₆-Alkyl, C₂₋₆-Alkenyl, C₂₋₆-Alkinyl, C₃₋₆-Cycloalkyl, Phenyl, Naphthyl, Heteroaryl, Heterocyclyl, C₃₋₆-Cycloalkyl-C₁₋₆-Alkyl-, Phenyl-C₁₋₆-Alkyl-, Naphthyl-C₁₋₆-Alkyl-, Heteroaryl-C₁₋₆-Alkyl- und Heterocyclyl-C₁₋₆-Alkyl- ausgewählt ist; wobei Heteroaryl ein 5-6-gliedriger Ring mit einem, zwei oder drei Heteroatomen ist, die jeweils unabhängig voneinander aus N, O und S ausgewählt sind; wobei Heteroaryl optional mit einem oder mehreren Substituenten substituiert ist, die jeweils unabhängig voneinander aus R^{b} ausgewählt sind; wobei Heterocyclyl ein 4-7-gliedriger Ring ist, der optional mit einem oder mehreren Substituenten substituiert ist, die jeweils unabhängig voneinander aus R^{c} ausgewählt sind; wobei, wenn Heterocyclyl eine Gruppe -NH- enthält, diese Gruppe -NH- optional mit R^{d} substituiert ist; wobei C₂₋₆-Alkenyl und C₂₋₆-Alkinyl jeweils unabhängig voneinander optional mit einem oder mehreren Substituenten substituiert sind, die jeweils unabhängig voneinander aus R^{e} ausgewählt sind, wobei C₁₋₆-Alkyl optional mit einem oder mehreren Substituenten substituiert ist, die jeweils unabhängig voneinander aus R^{f} ausgewählt sind; und wobei C₃₋₆-Cycloalkyl unabhängig voneinander optional mit einem oder mehreren Substituenten substituiert ist, die jeweils unabhängig voneinander aus R^{g} ausgewählt sind; und
R^{y} unabhängig aus Alkylsilyl, Arylsilyl und Heteroarylsilyl ausgewählt ist, wobei das Heteroaryl des Heteroarylsilyls ein 5-6-gliedriger Ring mit einem, zwei oder drei Heteroatomen ist, die jeweils unabhängig aus N, O und S ausgewählt sind, und optional mit einem oder mehreren Substituenten substituiert ist, die jeweils unabhängig aus R^{b} ausgewählt sind, und wobei das Alkyl oder Aryl des Alkylsilyls bzw. Arylsilyls optional mit einem oder mehreren Substituenten substituiert ist, die jeweils unabhängig aus R^{f} ausgewählt sind.

2. Verfahren gemäß Anspruch 1, wobei die Verbindung der Formel VIII oder ein Salz davon durch die folgenden Schritte hergestellt wird:
h) das Inkontaktbringen einer Verbindung der Formel VI oder eines Salzes davon: 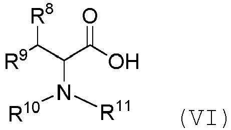 mit einem Aktivierungsreagenz, um eine Verbindung der Formel VII oder ein Salz davon zu bilden: 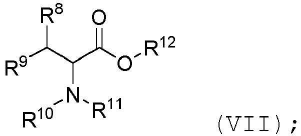 und
i) das Inkontaktbringen der Verbindung der Formel VII oder eines Salzes davon mit einem Amin, um die Verbindung der Formel VIII oder ein Salz davon herzustellen.

3. Verfahren gemäß Anspruch 1 oder 2, wobei
die Verbindung der Formel II oder ein Salz davon durch das Inkontaktbringen einer Verbindung der Formel I oder eines Salzes davon: 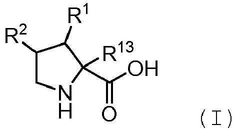
mit einem Aktivierungsreagenz und einem Alkohol hergestellt wird; und
die Verbindung der Formel III oder ein Salz davon durch das Inkontaktbringen der Verbindung der Formel II oder eines Salzes davon mit einem aktivierten Carbonylreagenz und einer Base hergestellt wird.

4. Verfahren gemäß Anspruch 2 oder 3, wobei das Aktivierungsreagenz SOCl₂ umfasst.

5. Verfahren gemäß Anspruch 3, wobei der Alkohol MeOH ist.

6. Verfahren gemäß Anspruch 3, wobei das aktivierte Carbonylreagenz Cbz-Cl ist.

7. Verfahren gemäß Anspruch 3, wobei die Base ein Hydroxidsalz ist.

8. Verfahren gemäß einem der vorhergehenden Ansprüche, wobei das Reagenz, das in der Lage ist, die Hydrolyse der Verbindung der Formel IV oder eines Salzes davon zu bewirken, LiOH umfasst.

9. Verfahren gemäß Anspruch 2, wobei das Amin NH₃ ist.

10. Verfahren gemäß einem der vorhergehenden Ansprüche, wobei das Aktivierungsreagenz in Schritt (c) 1-Ethyl-3-(3-dimethylaminopropyl)carbodiimid umfasst.

11. Verfahren gemäß Anspruch 1, wobei die aktivierte Carbonylverbindung Cbz-Cl ist.

12. Verfahren gemäß Anspruch 1, ferner umfassend das Inkontaktbringen der Verbindung der Formel VI oder eines Salzes davon mit einer Base.

13. Verfahren gemäß Anspruch 12, wobei die Base NaHCO₃ ist.

14. Verfahren gemäß einem der vorhergehenden Ansprüche, wobei das Carbamatspaltungsreagenz Palladium auf Kohlenstoff umfasst.

15. Verfahren gemäß einem der vorangehenden Ansprüche, wobei die Verbindung der Formel I 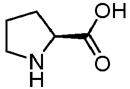 und/oder die Verbindung der Formel II 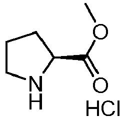 und/oder die Verbindung der Formel III 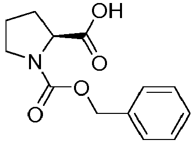 und/oder die Verbindung der Formel IV 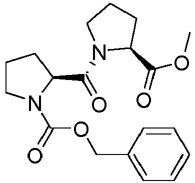 und/oder die Verbindung der Formel V 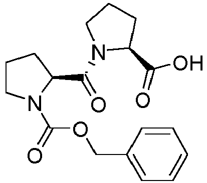 und/oder die Verbindung der Formel VI 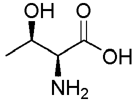 und/oder die Verbindung der Formel VII 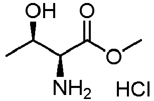 und/oder die Verbindung der Formel VIII 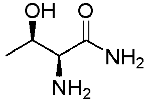 und/oder die Verbindung der Formel IX 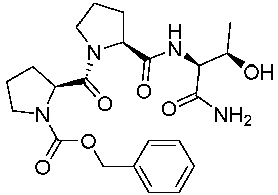 und/oder die Verbindung der Formel X 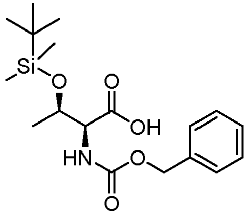 und/oder die Verbindung der Formel XI 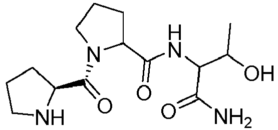 und/oder die Verbindung der Formel XIIa 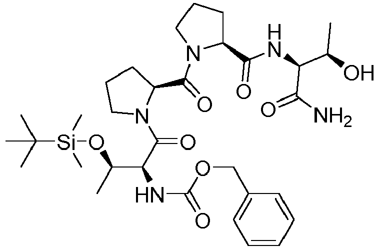 und/oder die Verbindung der Formel XIII 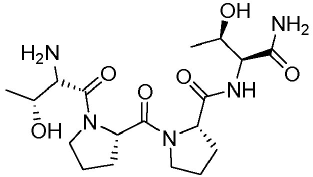 ist.

## Revendications

1. Procédé de synthèse d'un composée peptidique de dipyrrolidine ou d'un sel, stéréoisomère, métabolite, ou hydrate de celui-ci pharmaceutiquement acceptable, comprenant les étapes suivantes :
a) la mise en contact d'un composé de Formule III, ou d'un sel de celui-ci : 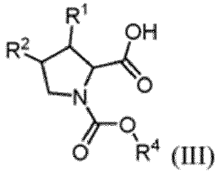 avec un réactif d'activation et un composé de Formule II, ou un sel de celui-ci : 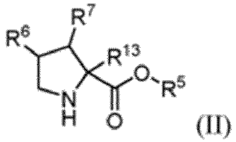 pour produire un composé de Formule IV, ou un sel de celui-ci : 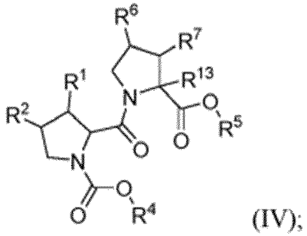
b) la mise en contact du composé de Formule IV, ou d'un sel de celui-ci, avec un réactif apte à effectuer une hydrolyse pour produire un composé de Formule V, ou un sel de celui-ci : 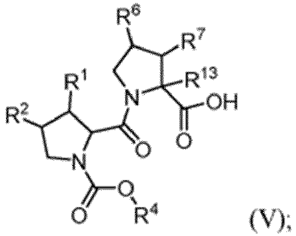
c) la mise en contact du composé de Formule V, ou d'un sel de celui-ci, avec un réactif d'activation et un composé de Formule VIII : 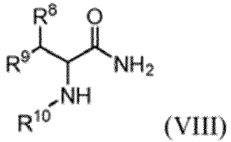 pour produire un composé de Formule IX, ou un sel de celui-ci : 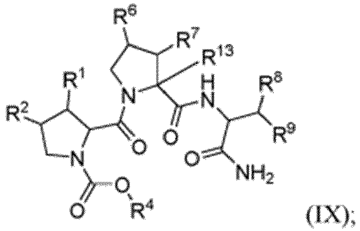
d) la mise en contact du composé de Formule IX, ou d'un sel de celui-ci, avec un réactif de clivage de carbamate pour produire un composé de Formule XI, ou un sel de celui-ci : 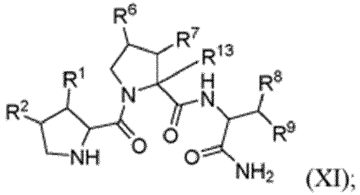
e) la mise en contact d'un composé de Formule X, ou d'un sel de celui-ci : 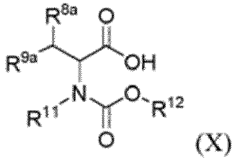 avec un réactif d'activation et le composé de Formule XI, ou un sel de celui-ci, pour produire un composé de Formule Xlla, ou un sel de celui-ci : 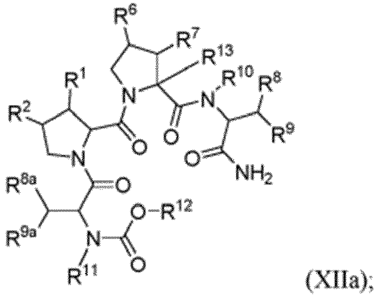
f) la mise en contact du composé de Formule Xlla, ou d'un sel de celui-ci, avec un réactif de clivage d'éther de silyle pour produire un composé de Formule XII, ou un sel de celui-ci : 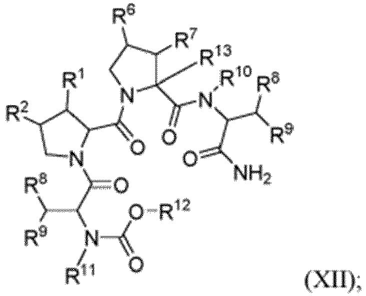 et
g) la mise en contact du composé de Formule XII, ou d'un sel de celui-ci, avec un réactif de clivage de carbamate pour produire un composé de Formule XIII, ou un sel de celui-ci : 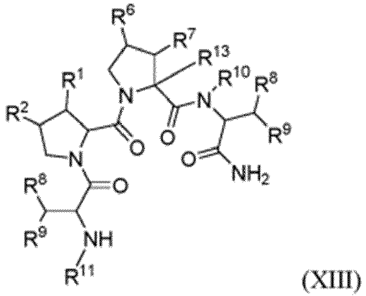 dans lequel le composé de Formule X, ou un sel de celui-ci, est produit par
x) la mise en contact d'un composé de Formule VI, ou d'un sel de celui-ci : 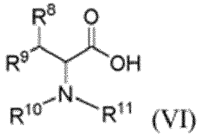 avec un composé carbonyle activé pour produire un composé de Formule Xa, ou un sel de celui-ci : 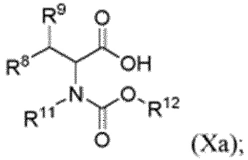 et
y) la mise en contact du composé de Formule Xa, ou d'un sel de celui-ci, avec un halogénure de silyle pour produire un composé de Formule X, ou un sel de celui-ci ;
dans lequel :
R¹ et R² sont indépendamment choisis parmi hydrogène, halogène, alkyle en C₁₋₆ substitué ou non substitué, alcoxy en C₁₋₆ substitué ou non substitué et aryle substitué ou non substitué, ou R¹ et R², conjointement avec les atomes auxquels ils sont fixés, forment un cycle hétérocyclique ou cycloalkyle de 4-6 chaînons substitué ou non substitué ;
R⁴, R⁵ et R¹² sont indépendamment -alkylène en C₁₋₆-phényle, dans lequel alkylène en C₁₋₆ est éventuellement substitué par un ou plusieurs substituants chacun indépendamment choisi parmi R^{f} ;
R⁶ et R⁷ sont indépendamment choisis parmi hydrogène, halogène, hydroxyle, alkyle en C₁₋₆ substitué ou non substitué, alcoxy en C₁₋₆ substitué ou non substitué et aryle substitué ou non substitué, ou R⁶ et R⁷, conjointement avec les atomes auxquels ils sont fixés, forment un cycle hétérocyclique ou cycloalkyle de 4-6 chaînons substitué ou non substitué ;
R⁸ et R⁹ sont indépendamment choisis parmi hydrogène, halogène, alkyle en C₁₋₆, alcényle en C₂₋₆, alcynyle en C₂₋₆, alcynyle en C₃₋₆, phényle, naphtyle, hétéroaryle, hétérocyclyle, cycloalkyle en C₃₋₆-alkyle en C₁₋₆-, phényle-alkylène en C₁₋₆-, naphtyle-alkylène en C₁₋₆-, hétéroaryle-alkylène en C₁₋₆-, hétérocyclyle-alkylène en C₁₋₆-, -OR^{X}, -NO₂, -N₃, -CN, -SCN, -SR^{X}, -C(O)R^{X}, -CO₂(R^{X}), -C(O)N(R^{X})₂, - C(NR^{X})N(R^{X})₂, -OC(O)R^{X}, -OCO₂R^{X}, - OC(O)N(R^{X})₂, -N(R^{X})₂, -SOR^{X}, -S(O)₂R^{X}, -NR^{X}C(O)R^{X}, -NR^{X}C(O)N(R^{X})₂, -NR^{X}C(O)OR^{X}, - NR^{X}C(NR^{X})N(R^{X})₂ et -C(R^{X})₃ ; dans lequel hétéroaryle est un cycle de 5-6 chaînons présentant un, deux, ou trois hétéroatomes chacun indépendamment choisi parmi N, O et S ; dans lequel hétéroaryle est éventuellement substitué par un ou plusieurs substituants chacun indépendamment choisi parmi R^{b} ; dans lequel hétérocyclyle est un cycle de 4-7 chaînons éventuellement substitué par un ou plusieurs substituants chacun indépendamment choisi parmi R^{c} ; dans lequel quand hétérocyclyle contient un fragment -NH-, ce fragment -NH- est éventuellement substitué par R^{d} ; dans lequel alcényle en C₂₋₆ et alcynyle en C₂₋₆ sont chacun indépendamment éventuellement substitué par un ou plusieurs substituants chacun indépendamment choisi parmi R^{e} ; dans lequel alkyle en C₁₋₆ et alkylène en C₁₋₆ sont chacun indépendamment éventuellement substitué par un ou plusieurs substituants chacun indépendamment choisi parmi R^{f} ; et dans lequel cycloalkyle en C₃₋₆ est indépendamment éventuellement substitué par un ou plusieurs substituants chacun indépendamment choisi parmi R^{g} ;
R^{8a} et R^{9a} sont indépendamment choisis parmi hydrogène, halogène, alkyle en C₁₋₆, alcényle en C₂₋₆, alcynyle en C₂₋₆, cycloalkyle en C₃₋₆, phényle, naphtyle, hétéroaryle, hétérocyclyle, cycloalkyle en C₃₋₆-alkyle en C₁₋₆-, phényle-alkylène en C₁₋₆-, naphtyle-alkylène en C₁₋₆-, hétéroaryle-alkylène en C₁₋₆-, hétérocyclyle-alkylène en C₁₋₆-, -OR^{X}, -OR^{y}, -NO₂, -N₃, -CN, -SCN, -SR^{X}, -C(O)R^{X}, -CO₂(R^{X}), -C(O)N(R^{X})₂, - C(NR^{X})N(R^{X})₂, -OC(O)R^{X}, - OCO₂R^{X}, -OC(O)N(R^{X})₂, -N(R^{X})₂, -SOR^{X}, -S(O)₂R^{X}, -NR^{X}C(O)R^{X}, -NR^{X}C(O)N(R^{X})₂, -NR^{X}C(O)OR^{X}, -NR^{X}C(NR^{X})N(R^{X})₂ et -C(R^{X})₃ ; dans lequel hétéroaryle est un cycle de 5-6 chaînons présentant un, deux, ou trois hétéroatomes chacun indépendamment choisi parmi N, O et S ; dans lequel hétéroaryle est éventuellement substitué par un ou plusieurs substituants chacun indépendamment choisi parmi R^{b} ; dans lequel hétérocyclyle est un cycle de 4-7 chaînons éventuellement substitué par un ou plusieurs substituants chacun indépendamment choisi parmi R^{c} ; dans lequel quand hétérocyclyle contient un fragment -NH-, ce fragment -NH- est éventuellement substitué par R^{d} ; dans lequel alcényle en C₂₋₆ et alcynyle en C₂₋₆ sont chacun indépendamment éventuellement substitué par un ou plusieurs substituants chacun indépendamment choisi parmi R^{e} ; dans lequel alkyle en C₁₋₆ et alkylène en C₁₋₆ sont chacun indépendamment éventuellement substitué par un ou plusieurs substituants chacun indépendamment choisi parmi R^{f} ; dans lequel cycloalkyle en C₃₋₆ est indépendamment éventuellement substitué par un ou plusieurs substituants chacun indépendamment choisi parmi R^{g} ; et dans lequel un de R^{8a} ou R^{9a} est - OR^{y}
R¹⁰ et R¹¹ sont indépendamment choisis parmi hydrogène, alkyle en C₁₋₆, -C(O)-alkylène en C₁₋₆, -C(O)-O-alkylène en C₁₋₆ et -C(O)-phényle, dans lequel alkyle en C₁₋₆, alkylène en C₁₋₆ et phényle sont éventuellement indépendamment substitués par un ou plusieurs substituants choisis parmi R^{a} ;
R¹³ est choisi parmi hydrogène et benzyle ;
R^{b} est choisi, indépendamment pour chaque occurrence, parmi halogène, hydroxyle, -NO₂, -N₃, -CN, -SCN, alkyle en C₁₋₆, alcényle en C₂₋₆, alcynyle en C₂₋₆, cycloalkyle en C₃₋₆, alcoxy en C₁₋₆, alcényloxy en C₃₋₆, alcynyloxy en C₃₋₆, cycloalcoxy en C₃₋₆, alkyle en C₁₋₆-S(O)_{w}-, où w est 0, 1, ou 2, alkyle en C₁₋₆-cycloalkyle en C₃₋₆-, cycloalkyle en C₃₋₆-alkyle en C₁₋₆-, alcoxycarbonyle en C₁₋₆-N(R^{a})-, alkyle en C₁₋₆-N(R^{a})-, alkyle en C₁₋₆-N(R^{a})carbonyle-, R^{a}R^{a'}N-, R^{a}R^{a}'N-carbonyle-, RaR^{a'}N-carbonyle-N(R^{a})-, R^{a}R^{a'}N-SO₂- et alkyle en C₁₋₆-carbonyle-N(R^{a})- ;
R^{a} et R^{a'} sont choisis, indépendamment pour chaque occurrence, parmi hydrogène et alkyle en C₁₋₆, ou R^{a} et R^{a'}, lorsqu'ils sont pris ensemble avec l'azote auquel ils sont fixés, forment un cycle hétérocyclique de 4-6 chaînons, dans lequel alkyle en C₁₋₆ est éventuellement substitué par un ou plusieurs substituants chacun indépendamment choisi parmi halogène, oxo et hydroxyle, et dans lequel le cycle hétérocyclique est éventuellement substitué par un ou plusieurs substituants chacun indépendamment choisi parmi halogène, alkyle, oxo et hydroxyle ;
R^{c} est choisi, indépendamment pour chaque occurrence, parmi halogène, hydroxyle, -NO₂, -N₃, -CN, -SCN, oxo, alkyle en C₁₋₆, alcényle en C₂₋₆, alcényle en C₂₋₆, cycloalkyle en C₃₋₆, alcoxy en C₁₋₆, alcényloxy en C₃₋₆, alcynyloxy en C₃₋₆, cycloalcoxy en C₃₋₆, alkyle en C₁₋₆-S(O)_{w}-, où w est 0, 1, ou 2, alkyle en C₁₋₆-cycloalkyle en C₃₋₆-, cycloalkyle en C₃₋₆-alkyle en C₁₋₆-, alcoxycarbonyle en C₁₋₆-N(R^{a})-, alkyle en C₁₋₆-N(R^{a})-, alkyle en C₁₋₆-N(R^{a})carbonyle-, R^{a}R^{a'}N-, R^{a}R^{a'}N-carbonyle-, R^{a}R^{a'}N-carbonyle-N(R^{a})-, R^{a}R^{a'}N-SO₂- et alkyle en C₁₋₆-carbonyle-N(R^{a})- ;
R^{d} est choisi, indépendamment pour chaque occurrence, parmi alkyle en C₁₋₆, alkylcarbonyle en C₁₋₆ et alkylsulfonyle en C₁₋₆, dans lequel alkyle en C₁₋₆ est éventuellement substitué par un ou plusieurs substituants chacun indépendamment choisi parmi halogène, hydroxyle et R^{a}R^{a'}N- ;
R^{e} est choisi, indépendamment pour chaque occurrence, parmi halogène, hydroxyle, -NO₂, -N₃, -CN, -SCN, alcoxy en C₁₋₄, alcoxycarbonyle en C₁₋₄, R^{a}R^{a'}N-, R^{a}R^{a}'N-carbonyle, R^{a}R^{a'}N-SO₂- et alkyle en C₁₋₄S(O)_{w}-, où w est 0, 1, ou 2 ;
R^{f} est choisi, indépendamment pour chaque occurrence, parmi halogène, hydroxyle, -NO₂, -N₃, -CN, -SCN, alcoxy en C₁₋₄, alcoxycarbonyle en C₁₋₄, R^{a}R^{a'}N-, R^{a}R^{a'}N-carbonyle, R^{a}R^{a'}N-SO₂- et alkyle en C₁₋₄ S(O)_{w}-, où w est 0, 1, ou 2 ;
R^{g} est choisi, indépendamment pour chaque occurrence, parmi halogène, hydroxyle, -NO₂, -N₃, -CN, -SCN, alkyle en C₁₋₆, alcoxy en C₁₋₄, alcoxycarbonyle en C₁₋₄, R^{a}R^{a'} N-, R^{a}R^{a'}N-carbonyle, R^{a}R^{a'}N- SO₂- et alkyle en C₁₋₄ S(O)_{w}-, où w est 0, 1, ou 2 ;
R^{X} est choisi, indépendamment, parmi hydrogène, halogène, alkyle en C₁₋₆, alcényle en C₂₋₆, alcynyle en C₂₋₆, cycloalkyle en C₃₋₆, phényle, naphtyle, hétéroaryle, hétérocyclyle, cycloalkyle en C₃₋₆-alkyle en C₁₋₆-, phényle-alkyle en C₁₋₆-, naphtyle-alkyle en C₁₋₆-, hétéroaryle-alkyle en C₁₋₆- et hétérocyclyle-alkyle en C₁₋₆- ; dans lequel hétéroaryle est un cycle de 5-6 chaînons présentant un, deux, ou trois hétéroatomes chacun indépendamment choisi parmi N, O et S ; dans lequel hétéroaryle est éventuellement substitué par un ou plusieurs substituants chacun indépendamment choisi parmi R^{b} ; dans lequel hétérocyclyle est un cycle de 4-7 chaînons éventuellement substitué par un ou plusieurs substituants chacun indépendamment choisi parmi R^{c} ; dans lequel quand hétérocyclyle contient un fragment -NH-, ce fragment -NH- est éventuellement substitué par R^{d} ; dans lequel alcényle en C₂₋₆ et alcynyle en C₂₋₆ sont chacun indépendamment éventuellement substitué par un ou plusieurs substituants chacun indépendamment choisi parmi R^{e} ; dans lequel alkyle en C₁₋₆ est éventuellement substitué par un ou plusieurs substituants chacun indépendamment choisi parmi R^{f} ; et dans lequel cycloalkyle en C₃₋₆ est indépendamment éventuellement substitué par un ou plusieurs subtituants chacun indépendamment choisi parmi R^{g} ; et
R^{y} est choisi, indépendamment, parmi alkylsyslile, arylsilyle et hétéroarylsilyle, dans lequel l'hétéroaryle d'hétéroarylsilyle est un cycle de 5-6 chaînons présentant un, deux, ou trois hétéroatomes chacun indépendamment choisi parmi N, O et S et éventuellement substitué par un ou plusieurs substituants chacun indépendamment choisi parmi R^{b} ; et dans lequel l'alkyle ou l'aryle de l'alkylsilyle ou de l'arylsilyle, respectivement, est éventuellement substitué par un ou plusieurs substituants chacun indépendamment choisi parmi R^{f}.

2. Procédé selon la revendication 1, dans lequel le composé de Formule VIII, ou un sel de celui-ci, est produit par les étapes suivantes :
h) la mise en contact d'un composé représenté par la Formule VI, ou d'un sel de celui-ci : 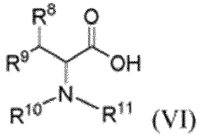 avec un réactif d'activation pour former un composé représenté par la Formule VII, ou un sel de celui-ci : 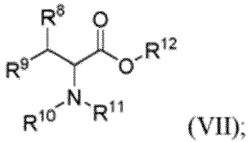 et
i) la mise en contact du composé de Formule VII, ou d'un sel de celui-ci, avec une amine pour produire le composé de Formule VIII, ou un sel de celui-ci.

3. Procédé selon la revendication 1 ou 2, dans lequel
le composé de Formule II, ou un sel de celui-ci, est produit par la mise en contact d'un composé de Formule I, ou d'un sel de celui-ci : 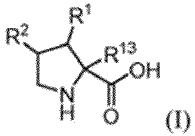
avec un réactif d'activation et un alcool ; et
le composé de Formule III, ou un sel de celui-ci, est produit par la mise en contact du composé de Formule II, ou d'un sel de celui-ci, avec un réactif carbonyle activé et une base.

4. Procédé selon la revendication 2 ou 3, dans lequel le réactif d'activation comprend SOCl₂.

5. Procédé selon la revendication 3, dans lequel l'alcool est MeOH.

6. Procédé selon la revendication 3, dans lequel le réactif carbonyle activé est Cbz-CI.

7. Procédé selon la revendication 3, dans lequel la base est un sel hydroxyde.

8. Procédé selon une quelconque revendication précédente, dans lequel le réactif apte à effectuer une hydrolyse du composé de Formule IV, ou d'un sel de celui-ci, comprend LiOH.

9. Procédé selon la revendication 2, dans lequel l'amine est NH₃.

10. Procédé selon une quelconque revendication précédente, dans lequel le réactif d'activation à l'étape (c) comprend du 1-éthyl-3-(3-diméthylaminopropyl)carbodiimide.

11. Procédé selon la revendication 1, dans lequel le composé carbonyle activé est Cbz-Cl.

12. Procédé selon la revendication 1, comprenant en outre la mise en contact du composé de Formule VI, ou d'un sel de celui-ci, avec une base.

13. Procédé selon la revendication 12, dans lequel la base est NaHCO₃.

14. Procédé selon une quelconque revendication précédente, dans lequel le réactif de clivage de carbamate comprend du palladium sur carbone.

15. Procédé selon une quelconque revendication précédente, dans lequel le composé de Formule I est 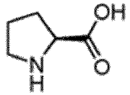 et/ou le composé de Formule II est 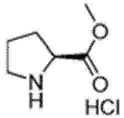 et/ou le composé de Formule III est 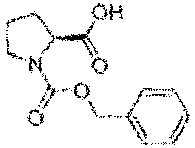 et/ou le composé de Formule IV est 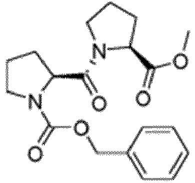 et/ou le composé de Formule V est 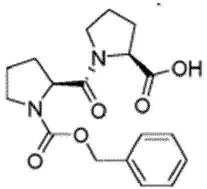 et/ou le composé de Formule VI est 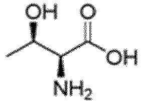 et/ou le composé de Formule VII est 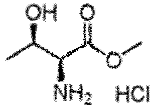 et/ou le composé de Formule VIII est 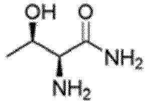 et/ou le composé de Formule IX est 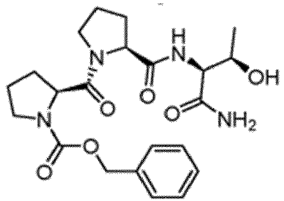 et/ou le composé de Formule X est 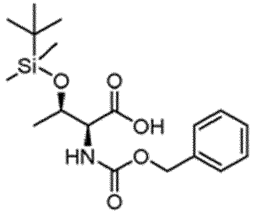 et/ou le composé de Formule XI est 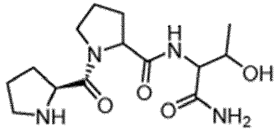 et/ou le composé de Formule Xlla est 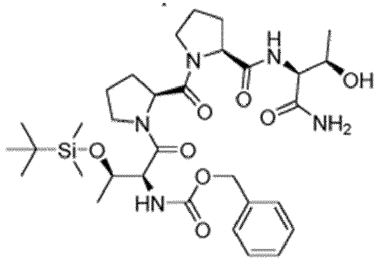 et/ou le composé de Formule XIII est
